# EUROPEAN PATENT SPECIFICATION

(11) **EP 3 486 322 B1**
(45) Date of publication and mention of the grant of the patent: **29.09.2021**
(21) Application number: 17827693.7
(22) Date of filing: 13.07.2017
(51) Int. Cl.: C12N 15/09, A61K 38/00, A61K 48/00, A61P 35/00, C12N 7/01

(54) **METHOD FOR PRODUCING VACCINIA VIRUS EXPRESSING FOREIGN GENE**
VERFAHREN ZUR HERSTELLUNG EINES EIN FREMDES GEN EXPRIMIERENDEN VACCINIA VIRUS
PROCÉDÉ DE FABRICATION DE VIRUS VACCINAL EXPRIMANT UN GÈNE ÉTRANGER

(30) Priority: 13.07.2016 JP 2016138712
(43) Date of publication of application: 22.05.2019
(73) Proprietor: National University Corporation Tottori University, Tottori-shi, Tottori 680-8550 (JP)
(72) Inventor: NAKAMURA, Takafumi, Yonago-shi Tottori 683-8503 (JP); NAKATAKE, Motomu, Yonago-shi Tottori 683-8503 (JP); KUROSAKI, Hajime, Yonago-shi Tottori 683-8503 (JP); HORITA, Kousuke, Yonago-shi Tottori 683-8503 (JP)
(74) Representative: Wohlfahrt, Jan Günther
(86) International application number: PCT/JP2017/025486
(87) International publication number: WO 2018/012570

(56) References cited:
- WO-A1-02/090554
- WO-A1-2015/076422
- WO-A1-2016/008976
- WO-A1-2016/009017
- JP-A- 2001 204 474
- JP-A- 2008 519 024
- SÓNIA DUARTE ET AL: "Suicide gene therapy in cancer: Where do we stand now?", CANCER LETTERS, vol. 324, no. 2, 1 November 2012 (2012-11-01), pages 160-170, XP055518019, US ISSN: 0304-3835, DOI: 10.1016/j.canlet.2012.05.023
- ERBS P ET AL: "Modified vaccinia virus Ankara as a vector for suicide gene therapy", CANCER GENE THERAPY, APPLETON & LANGE, GB, vol. 15, no. 1, 9 November 2007 (2007-11-09), pages 18-28, XP002524702, ISSN: 0929-1903, DOI: 10.1038/SJ.CGT.7701098 [retrieved on 2007-11-09]
- MCCART J A ET AL: "Systemic cancer therapy with a tumor-selective vaccinia virus mutant lacking thymidine kinase and vaccinia growth factor genes", CANCER RESEARCH, AMERICAN ASSOCIATION FOR CANCER RESEARCH, US, vol. 61, no. 24, 15 December 2001 (2001-12-15), pages 8751-8757, XP002767885, ISSN: 0008-5472
- GOTO IKUMI: "177. Deletions of Both Vaccinia Growth Factor and O1 Protein Genes Enhance Therapeutic Index of Oncolytic Vaccinia Virus", MOLECULAR THERAPY : THE JOURNAL OF THE AMERICAN SOCIETY OF GENE THERAPY, vol. 22, 1 May 2014 (2014-05-01), pages S67-S68, XP055653727, US ISSN: 1525-0016, DOI: 10.1016/S1525-0016(16)35190-5

### SEQUENCE LISTING

<110> National University Corporation Tottori University
<120> A method for producing a vaccinia virus which expresses foreign genes
<130> PH-6983-PCT
<150> JP 2016-138712
   <151> 2016-07-13
<160> 11
<170> PatentIn version 3.5
<210> 1
   <211> 37
   <212> DNA
   <213> Artificial
<220>
   <223> Primer
<400> 1
   atggccggac cggccaccgg tcgccaccat gagcgag 37
<210> 2
   <211> 41
   <212> DNA
   <213> Artificial
<220>
   <223> Primer
<400> 2
   tcgaattcgc tagcggccgc ttaattaagc ttgtgcccca g 41
<210> 3
   <211> 20
   <212> DNA
   <213> Artificial
<220>
   <223> Primer
<400> 3
   ggtaacgcta tcgaaacgac 20
<210> 4
   <211> 20
   <212> DNA
   <213> Artificial
<220>
   <223> Primer
<400> 4
   ttagttcgtc gagtgaacct 20
   <210> 5
   <211> 20
   <212> DNA
   <213> Artificial
<220>
   <223> Primer
<400> 5
   acagggatta agacggaaag 20
<210> 6
   <211> 20
   <212> DNA
   <213> Artificial
<220>
   <223> Primer
<400> 6
   gtcaacaagc atcttccaac 20
<210> 7
   <211> 40
   <212> DNA
   <213> Artificial
<220>
   <223> Primer
<400> 7
   gcgctagcgg ccggaccggc catggtcaca ggaggcatgg 40
<210> 8
   <211> 27
   <212> DNA
   <213> Artificial
<220>
   <223> Primer
<400> 8
   gcacgcgttt agacacagta gtatctg 27
<210> 9
   <211> 3082
   <212> DNA
   <213> Artificial
<220>
   <223> Synthetic
<400> 9
   accggtcgcc accatggacc cggtggtgct gcagaggcgg gattgggaga atcctggggt      60
   gacgcagctg aatcggctgg ctgctcaccc accatttgca tcatggagaa attccgaaga     120
   ggcccggacc gaccgcccct ctcagcagct cagaagtctt aatggagaat ggcgcttcgc     180
   atggtttcct gctcccgagg ctgtaccgga aagttggctc gagtgcgatt tgcccgaggc 240
   agataccgtc gtggttccct ccaactggca gatgcacggc tatgatgccc ctatctacac 300
   caatgtcact taccctataa cagtgaaccc accctttgtg cctaccgaga atcccaccgg 360
   atgctacagt ctgacattta acgtggacga gtcttggctg caggaaggcc agactagaat 420
   catcttcgat ggtgtcaaca gcgcttttca tctgtggtgc aacgggcgtt gggtgggtta 480
   cggccaagac agtaggctcc cttctgaatt cgatctctct gccttcctgc gggccggtga 540
   gaatagactt gccgttatgg ttctgcgttg gagcgacggt tcctacctgg aggaccagga 600
   tatgtggagg atgtctggca ttttccgaga tgtgagcctc cttcacaaac ctaccactca 660
   aatctccgac tttcatgttg ccacaaggtt caacgacgac ttttcacgcg ctgttctgga 720
   ggccgaggtc caaatgtgcg gcgaactgcg cgattatctg cgcgtgactg tgagcctttg 780
   gcaaggagag acacaggtgg catcaggcac cgcacccttc ggcggagaaa tcatcgacga 840
   acggggagga tatgctgata gggttactct taggctgaat gtagaaaacc ccaagctctg 900
   gtctgcagaa atacctaacc tctatcgcgc agttgtggaa ctgcacacgg cagacgggac 960
   cctgattgaa gccgaagcct gtgacgtcgg cttccgtgaa gtgcgcatcg agaatgggct 1020
   gctccttctt aacggtaagc cactgttgat cagaggcgtg aataggcatg agcatcatcc 1080
   gctccacgga caggtgatgg atgagcagac aatggttcag gacatactct tgatgaaaca 1140
   gaacaacttc aatgccgtgc gctgtagcca ctaccctaat cacccactgt ggtataccct 1200
   gtgtgacagg tacggcctgt atgtcgtgga tgaggcaaac attgaaactc atggcatggt 1260
   gccaatgaat cggctgacag atgaccccag atggctgccc gccatgtcag agcgtgtgac 1320
   caggatggta cagcgggaca gaaatcaccc cagtgtcata atctggtccc ttgggaacga 1380
   atcagggcat ggtgcaaacc acgatgctct gtaccgctgg attaagagcg ttgaccctag 1440
   tcggccagtg cagtatgaag gtggaggcgc cgataccact gcaactgaca ttatttgccc 1500
   aatgtacgct cgggtcgacg aggatcaacc gttccctgcg gtcccaaagt ggagcattaa 1560
   gaaatggctg tctttgcctg gagaaacacg cccgctgatt ctgtgcgaat atgcccacgc 1620
   aatggggaac tccctgggcg ggtttgcaaa gtattggcag gcttttcgcc agtatccacg 1680
   actgcaggga ggctttgtgt gggactgggt agatcagagc ctgatcaaat acgacgaaaa 1740
   tggcaatcca tggtccgcct atggaggtga ctttggtgat acccctaatg acaggcagtt 1800
   ttgcatgaac ggactcgtct ttgcagatcg aactccacat ccggccctga ctgaggccaa 1860
   gcatcagcag caattcttcc agtttcggct gtctgggcag accattgagg tgacttccga 1920
   gtacttgttt cgacacagcg acaatgagct gctgcactgg atggtggccc tcgatggcaa 1980
   accactggcc tcaggagagg tgcccctgga tgtagcgccc caggggaaac agcttatcga 2040
   gttgcccgaa ctgccccaac ccgagtctgc tgggcaactc tggcttaccg tgcgagtcgt 2100
   tcagccaaat gccactgcct ggtccgaggc tggccacatt agcgcatggc agcagtggag     2160
   actggctgag aacctcagcg ttacccttcc cgcagcctct cacgccatcc ctcacttgac     2220
   cactagtgag atggacttct gtatcgagct gggcaacaaa cgctggcagt ttaacagaca     2280
   gtcaggcttc ttgtcccaga tgtggattgg cgacaagaag cagctgttga cccctttgcg     2340
   ggatcagttc acaagggcgc ctctggacaa tgacatcgga gtgagcgagg ctacacgaat     2400
   agatccaaac gcgtgggtcg agaggtggaa ggcggctggg cactaccaag ctgaagcggc     2460
   cctgttgcaa tgtaccgccg atacgctcgc cgatgccgtc ctcattacga cagcccacgc     2520
   ttggcagcac cagggcaaaa cactgtttat ctcccgtaag acatacagaa tcgatggcag     2580
   cggtcaaatg gccattacgg tagacgtgga agttgcgtca gatacacccc atcccgcgag     2640
   gatcggactg aactgtcaat tggcccaagt cgcagagaga gtgaactggc tgggactcgg     2700
   gcctcaggag aattatccag accggctcac agccgcttgc ttcgataggt gggaccttcc     2760
   actctctgat atgtacaccc catacgtgtt cccctcagag aatggcctgc ggtgtgggac     2820
   acgagaactg aactacggac cgcatcagtg gagaggggac ttccagttca acatcagccg     2880
   gtatagtcag cagcagctga tggaaacgtc ccatagacat ctgctgcacg ctgaggaagg     2940
   gacatggctg aacattgacg ggttccacat gggaataggt ggcgatgaca gctggtcccc     3000
   tagcgtaagc gccgagtttc aactgagtgc tgggagatat cattaccaac tggtctggtg     3060
   ccagaaatga gctagcgaat tc                                              3082
<210> 10
   <211> 423
   <212> DNA
   <213> Vaccinia virus
<400> 10
   atgttgataa attatctgat gttgttgttc gctgctatga taatcagatc attcgccgat       60
   agtggtaacg ctatcgaaac gacattgcca gaaattacaa acgctacaac agatattcca      120
   gctatcagat tatgcggtcc agagggagat ggatattgtt tacacggtga ctgtatccac      180
   gctagagata ttgacggtat gtattgtaga tgctctcatg gttatacagg cattagatgt      240
   cagcatgtag tattagtaga ctatcaacgt tcagaaaacc caaacactac aacgtcatat      300
   atcccatctc ccggtattat gcttgtatta gtaggcatta ttattattac gtgttgtcta      360
   ttatctgttt ataggttcac tcgacgaact aaactactta tacaagatat ggttgtgcca      420
   taa                                                                   423
<210> 11
   <211> 2001
   <212> DNA
   <213> Vaccinia virus
<400> 11
   atgttcatgt atccggaatt tgcgaggaag gctttatcaa agcttatttc aaaaaaatta 60
   aacattgaaa aggtgtctag caagcaccag ctcgtgttac tggattatgg attacacgga 120
   ctattgccaa aatcactgta tctggaagct attaactccg acattctcaa tgttaggttt 180
   tttcctcctg aaataataaa cgtcactgat atcgttaagg ctctccaaaa ttcttgtaga 240
   gtagatgagt acctaaaagc tgtttcctta tatcataaga attctttaat ggtatcggga 300
   ccaaatgtag tcaagcttat gatagaatat aatcttctta cacacagtga cttggaatgg 360
   ttaattaatg agaatgtagt caaggctaca taccttttaa aaatcaatgc ctatatgatt 420
   aactttaaaa tagatctaac ggttgatgaa atcattgact tagttaaaga tattcctgta 480
   ggagctacgc tacatctata taatatatta aacaatatag atttggacat tgttcttcgt 540
   atatctgatg aatataatat accacctgtt cacgatattc tgtctaaact taccgatgaa 600
   gagatgtgta taaaactagt tacaaagtat cctatggaca atgttataaa ttttattaat 660
   caagatgtta gatatagtcc caccttcatc aagacaatta aagattttgt caacaagcat 720
   cttccaacca tgtacgatgg attaaatgat tatctacatt ctgttattat cgacgaggac 780
   ttaatagagg aatataaaat taaatccgtt gccatgttta atttggaata caaaactgat 840
   gtaaatactc taacattgga cgaacagata tttgtagagg taaacatctc atattatgat 900
   tttagatata gacaatttgc cgatgaattt agagattaca ttatgataaa agaaagaaga 960
   caaatcacca tgcaatctgg tgatagaata agaaggttta gacgtcccat gtcattgaga 1020
   tccactatca tcaaaaagga tactgattct ctagaggata ttctcgcaca tatagataat 1080
   gccagaaaaa atagcaaggt atccattgaa gatgttgaga gaatcatttc atctttccgt 1140
   cttaatccct gtgttgtcag acgcaccatg ctgtctgata tagatatcaa aacaaagata 1200
   atggtgctaa aaattgtcaa agattggaaa tcttgtgctc tgacactatc agccatcaaa 1260
   ggaattatgg taacagatac catcaatacc gtgttatcca aaattctgca tcatcatagg 1320
   aatgtcttca agtatcttac atctgtagag aataaagaaa ttgctgtctg taattgctcc 1380
   agatgtctgt cgctcttcta tagagaatta aaaagtgtac gatgtgatct acacacagac 1440
   gatggattat tggataggct atacgatctg actagatacg ccttacacgg aaaaatcaat 1500
   caaaacttaa tcggtcaacg atgttggggt ccgttgacag aaatgctgtt taacgagaat 1560
   aaaaagaaaa aactaaataa tttaatggaa tacatcaaaa tatcagacat gttggtatac 1620
   ggacactcta tcgagaagac gcttattcca attactgatt ctctttcatt caagctatct 1680
   gttgatacca tgtctgtgtt aaatgatcaa tatgccaagg ttgtcatctt cttcaatacc 1740
   atcatagaat atattatagc tactatctat tatagattga cagtcttgga caattatact 1800
   aatgtcaaac attttgtatc caaagtgtta cacactgtca tggaagcatg tggcgtactg 1860
   ttttcataca ttaaagttaa tgacaaaata gagcatgaat tggaggagat ggtggacaaa     1920
   ggtaccgtac cttcttattt gtatcatctg tccatcaacg tcatttcaat aatattggat     1980
   gatataaatg gaactcgtta a                                               2001

## Description

### Technical Field

The present invention relates to a method for introducing a foreign gene to a vaccinia virus, a vaccinia virus having suicide genes and the vaccinia virus for use in treating cancer.

### Background Art

Currently, preclinical and clinical studies on cancer therapies using live viruses have been actively conducted worldwide. The cancer virus therapy is a method utilizing inherent nature of viruses, which is to kill infected cells and tissues by growing and propagating therein. Such a method, compared to the conventional radiation therapy and chemotherapy, exhibits anticancer effects via diverse mechanisms by firstly oncolysis due to virus growth, and secondly induction of antitumor immunity associated therewith.

There are vaccine strains of a vaccinia virus which were established in Japan some time ago and have been used in human as a smallpox vaccine and thereby proven to be highly safe (see Non Patent Literature 1). However, the strains still retain attenuated growth potential in normal tissues, and thus the modification for growing only in cancer cells was needed to be established as a safer cancer virus therapy. Thus, the modification was made based on these vaccine strains by gene recombination technique, and a gene recombinant vaccinia virus growing in and damaging specifically cancer cells was successfully developed, using control dysfunction of the MAPK/ERK pathway in a wide range of cancers as the indicator (see Patent Literatures 1 and 2). Patent Literature 3 and 4 disclose vaccinia viruses comprising suicide genes and a disrupted VGF gene. Non Patent Literature 2 and 3 describe the use of vaccinia viruses for suicide gene therapy. Non Patent Literature 4 discloses the introduction of EGFP into the VGF gene of vaccine viruses by homologous recombination. In Non Patent Literature 5, the effect of a combined deletion of VGF and OIL on safety profile, tumor specificity and therapeutic index of oncolytic vaccinia viruses has been investigated. Patent Literature 5 discloses the use of suicide genes in vaccinia viruses for cancer therapy. Patent Literature 6 discloses inactivation of VGF and/or OIL for increasing tumor specificity.

### Citation List

### Patent Literature

Patent Literature 1: International Publication No. WO2011/125469
Patent Literature 2: International Publication No. WO2015/076422
Patent Literature 3: International Publication No. WO2016/008976 A1
Patent Literature 4: International Publication No. WO2016/009017 A1
Patent Literature 5: Japanese Publication No. JP 2008 519024 A
Patent Literature 6: International Publication No. WO2015/076422 A1

### Non Patent Literature

Non Patent Literature 1: PROTEIN, NUCLEIC ACID AND ENZYME Vol. 48 No. 12 (2003), p.1693-1700
Non Patent Literature 2: CANCER LETTERS, Vol. 324, No. 2 (2012), p. 160-170
Non Patent Literature 3: CANCER GENE THERAPY, APPLETON & LANGE, Vol. 15, No. 1 (2007), p. 18-28
Non Patent Literature 4: CANCER RESEARCH, AMERICAN ASSOCIATION FOR CANCER RESEARCH, Vol. 61, No. 24 (2001), p. 8751-8757
Non Patent Literature 5: MOLECULAR THERAPY: THE JOURNAL OF THE AMERICAN SOCIETY OF GENE THERAPY, Vol. 22 (2014), p. S67-S68.

### Summary of Invention

### Technical Problem

The present invention has an object to provide a method for introducing a foreign gene to a vaccinia virus and the vaccinia virus for use in treating cancer.

### Solution to Problem

Vaccinia virus can be used as a cancer therapeutic virus by introducing a therapeutic gene as a foreign gene, and can also be used as a vaccine by introducing an antigen of a bacterium and the like as a foreign gene, and thereby a number of applications are conceivable. For these applications, a foreign gene needs to be introduced into the vaccinia virus, and the present inventors extensively conducted studies on methods for easily and quickly introducing a foreign gene to the vaccinia virus.

The present inventors first inserted a marker gene such as a fluorescent marker gene into an endogenous gene or an untranslated region of a vaccinia virus, and subsequently replaced the fluorescent marker gene with a foreign gene of interest to be introduced by homologous recombination. In the case of introducing a gene into a cell or a microorganism, the introduction of the gene is not easily confirmed, whereas in the case of inserting a marker gene first and subsequently replacing the marker gene with a foreign gene of interest, the introduction of the foreign gene can be confirmed using a loss of a signal such as fluorescence caused by the marker gene as an indicator, enabling easy, definite, and quick introduction of the foreign gene.

The present inventors inserted a fluorescent protein gene in advance to an endogenous VGF gene and an OIL gene of a vaccinia virus to prepare a vaccinia virus mitogen-activated protein kinase (MAPK)-dependent recombinant vaccinia virus (MD-RVV) being deprived of the VGF gene and the OIL gene and having an anticancer action (International Publication No. WO2015/076422). Suicide genes (CD gene and UPRT gene) were introduced by the above method to the vaccinia virus wherein the fluorescent protein gene was inserted into the endogenous gene. Such a vaccinia virus being deprived of the functions of the VGF gene and the OIL gene and further having the suicide genes inserted is confirmed to be capable of killing cancer cells by not only the effect of MD-RVV but also the effect of the suicide genes.

The present invention is defined by the claims. Any subject-matter falling outside the scope of the claims is provided for information purposes only.

### Advantageous Effects of Invention

A foreign gene of interest can be easily, definitely, and quickly introduced into a vaccinia virus when a marker gene such as a fluorescent marker gene is first inserted into an endogenous gene or an untranslated region of the vaccinia virus, and subsequently the fluorescent marker gene is replaced with the foreign gene of interest to be introduced by homologous recombination. Further, the vaccinia virus wherein a suicide gene prepared by this method is introduced can kill cancer cells by not only the cancer cell killing effect provided by the vaccinia virus but also the action of the suicide gene.

### Brief Description of Drawings

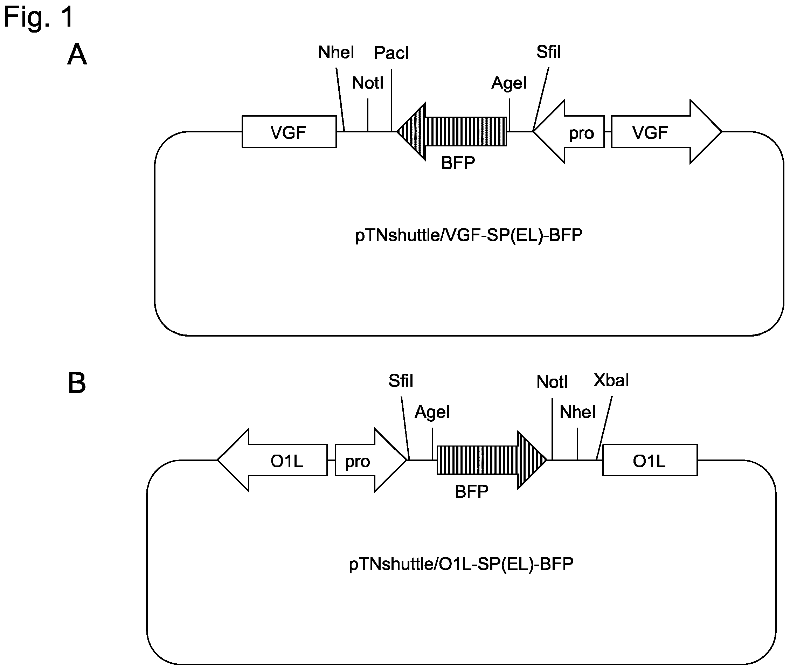

[Figure 1] Figures show the structures of shuttle vectors for inserting a foreign gene expression unit into the VGF or the OIL gene for generating a gene recombinant vaccinia. Figure 1A shows the structure of pTNshuttle/VGF-SP(EL)-BFP, and Figure 1B shows the structure of pTNshuttle/O1L-SP(EL)-BFP.

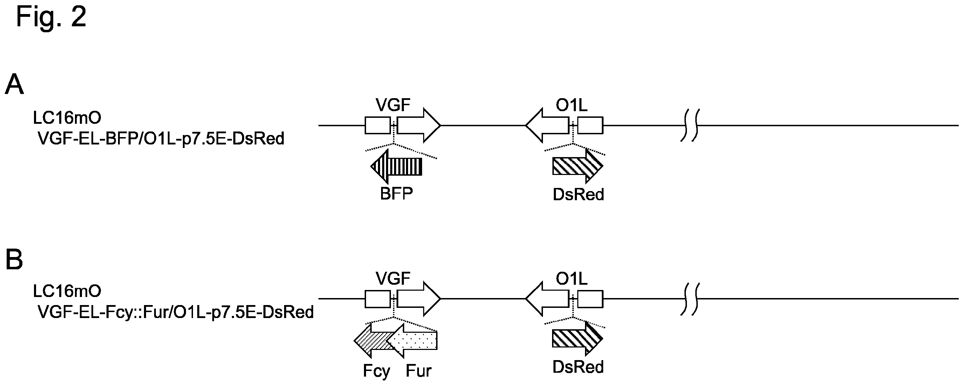

[Figure 2] Figures show the structures of recombinant vaccinia viruses LC16mO VGF-EL-BFP/O1L-p7.5E-DsRed (Figure 2A) and LC16mO VGF-EL-Fcy::Fur/O1L-p7.5E-DsRed (Figure 2B).

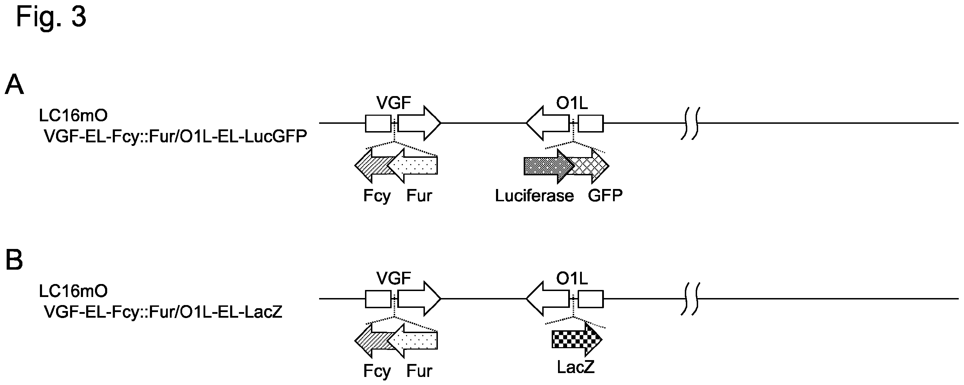

[Figure 3] Figures show the structures of recombinant vaccinia viruses LC16mO VGF-EL-Fcy::Fur/O1L-EL-LucGFP (Figure 3A) and VGF-EL-Fcy::Fur/O1L-EL-LacZ (Figure 3B).

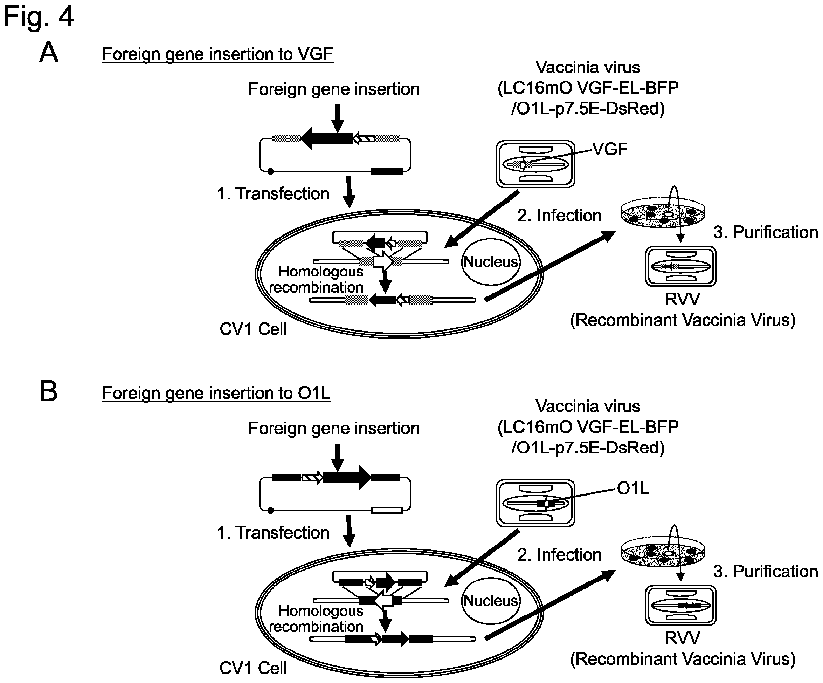

[Figure 4] Figures show the method for introducing a foreign gene by homologous recombination into the vaccinia virus wherein a florescent marker gene is inserted into the VGF gene (Figure 4A) and the OIL gene (Figure 4B).

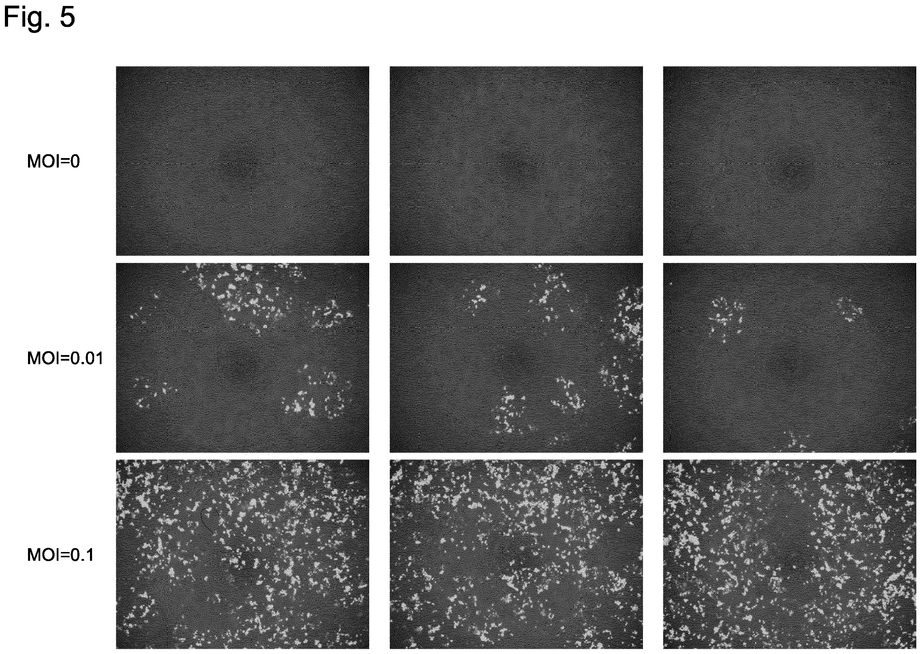

[Figure 5] Figure shows observed images for the relation of amount of vaccinia virus infected and GFP fluorescence intensity in Panc1 cells infected with LC16mO VGF-EL-Fcy::Fur/O1L-EL-LucGFP.

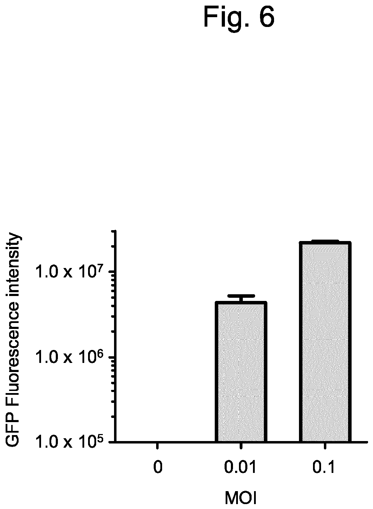

[Figure 6] Figure shows a quantified data for the relation of the amount of vaccinia virus infected and GFP fluorescence intensity in Panc1 cells infected with LC16mO VGF-EL-Fcy::Fur/O1L-EL-LucGFP.

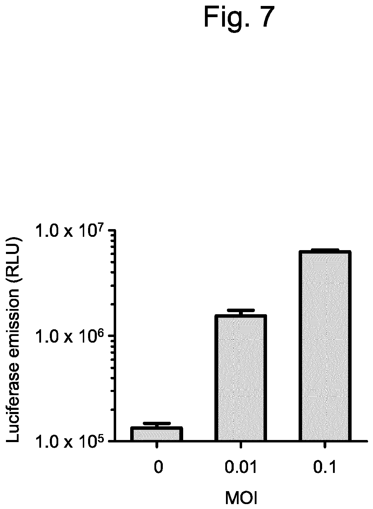

[Figure 7] Figure shows a quantified data for the relation of the amount of vaccinia virus infected and luciferase emission intensity in Panc1 cells infected with LC16mO VGF-EL-Fcy::Fur/O1L-EL-LucGFP.

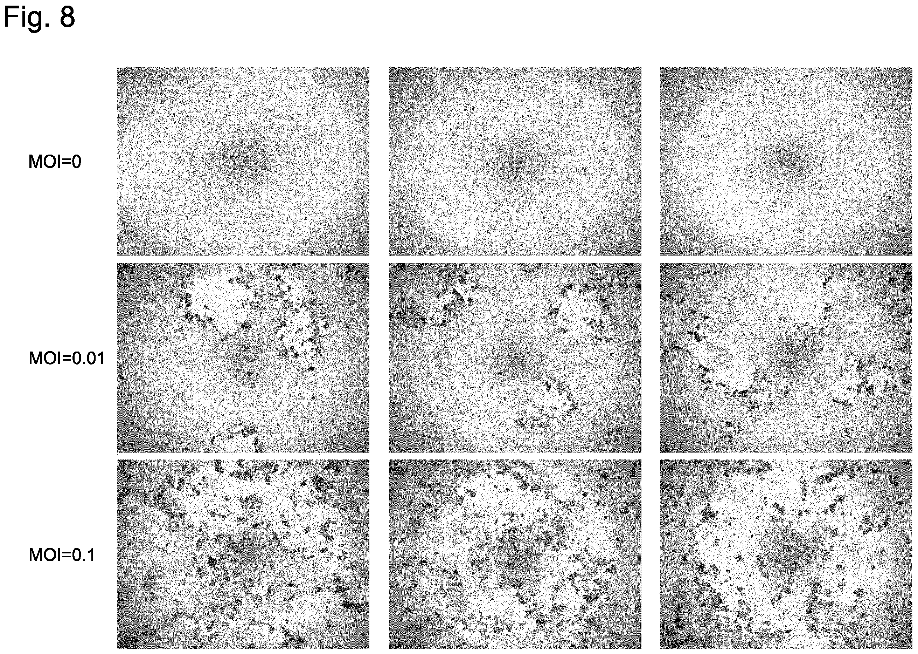

[Figure 8] Figure shows the results of staining Panc1 cells infected with LC16mO VGF-EL-Fcy::Fur/O1L-EL-LacZ using Genlatis X-Gal Staining Assay Kit.

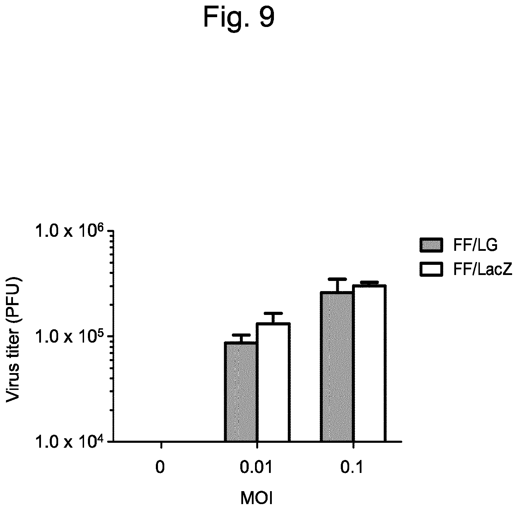

[Figure 9] Figure shows the virus titers in Panc1 cells infected with LC16mO VGF-EL-Fcy::Fur/O1L-EL-LucGFP (FF/LG) and LC16mO VGF-EL-Fcy::Fur/O1L-EL-LacZ (FF/LacZ).

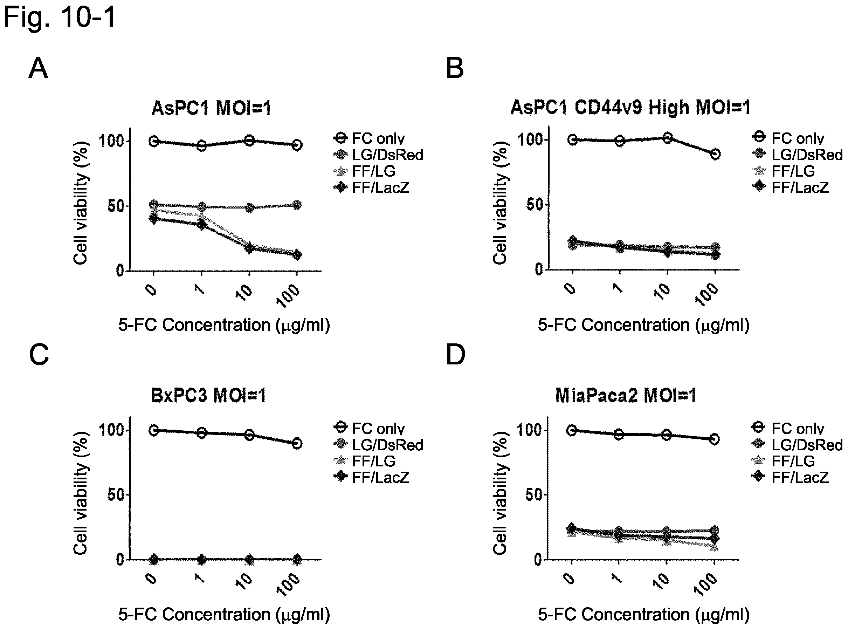

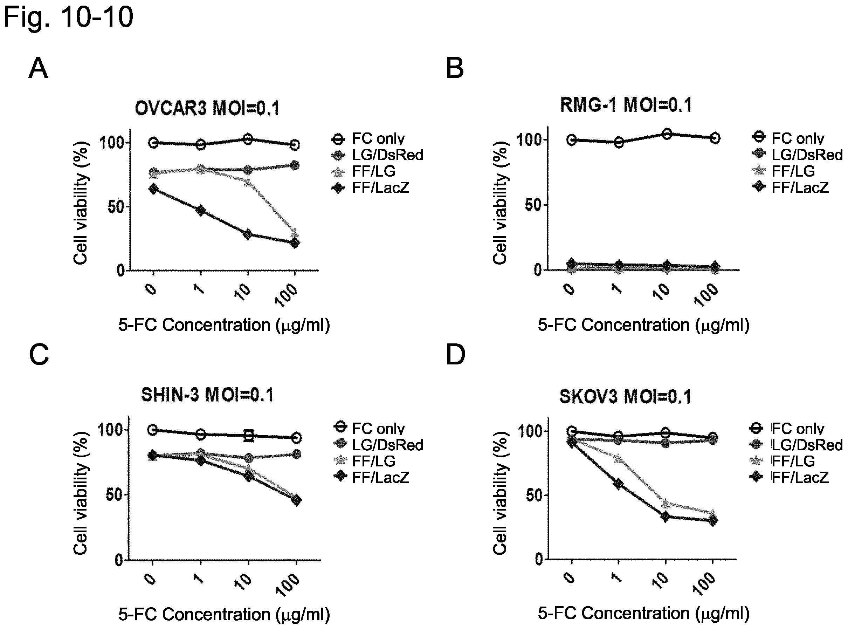

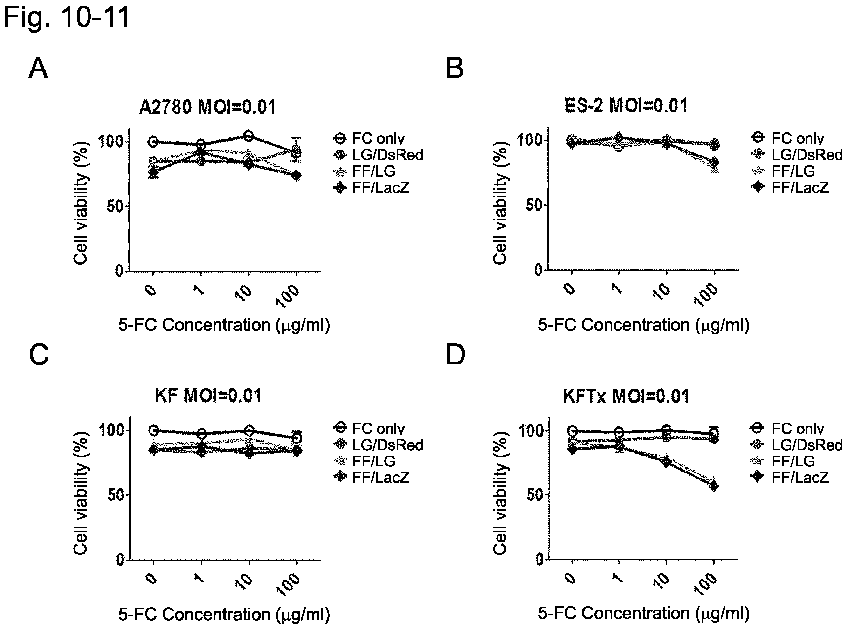

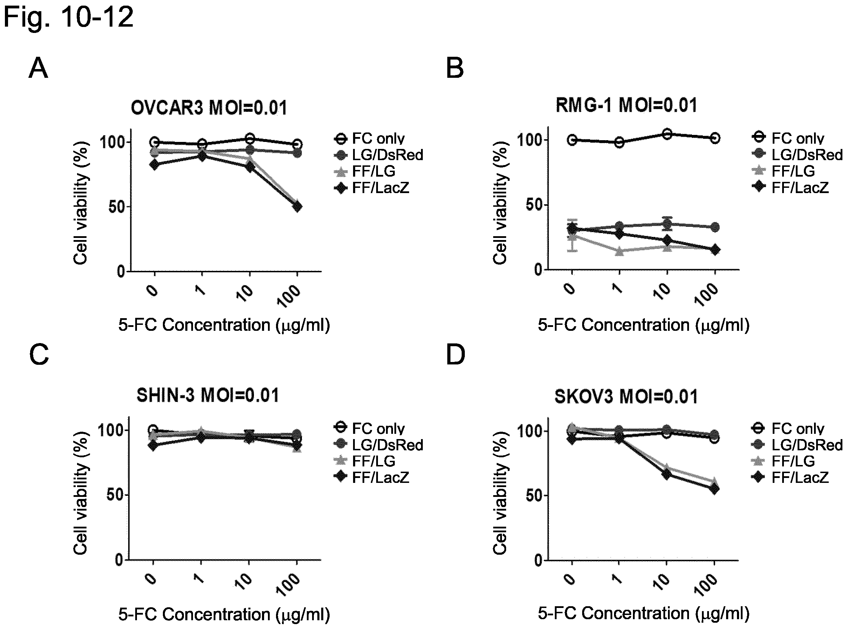

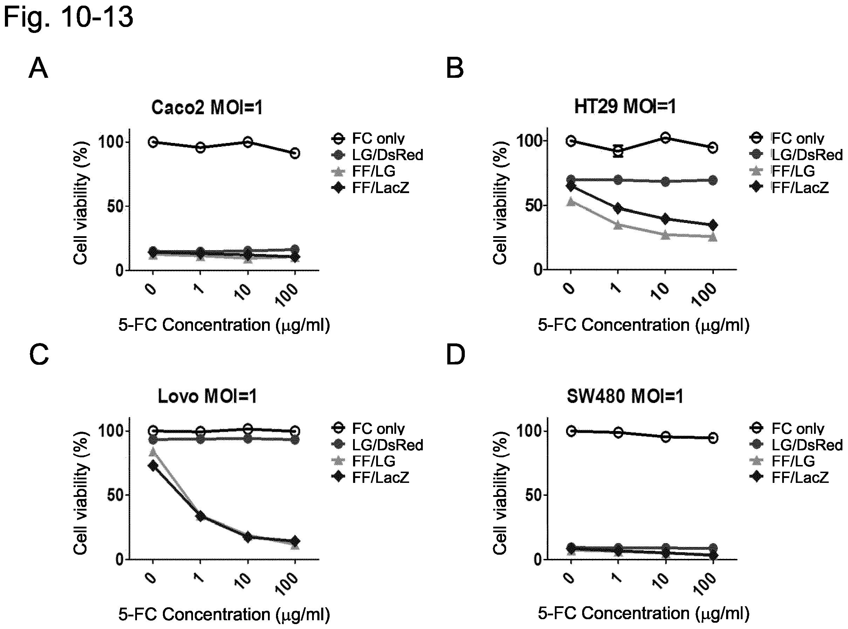

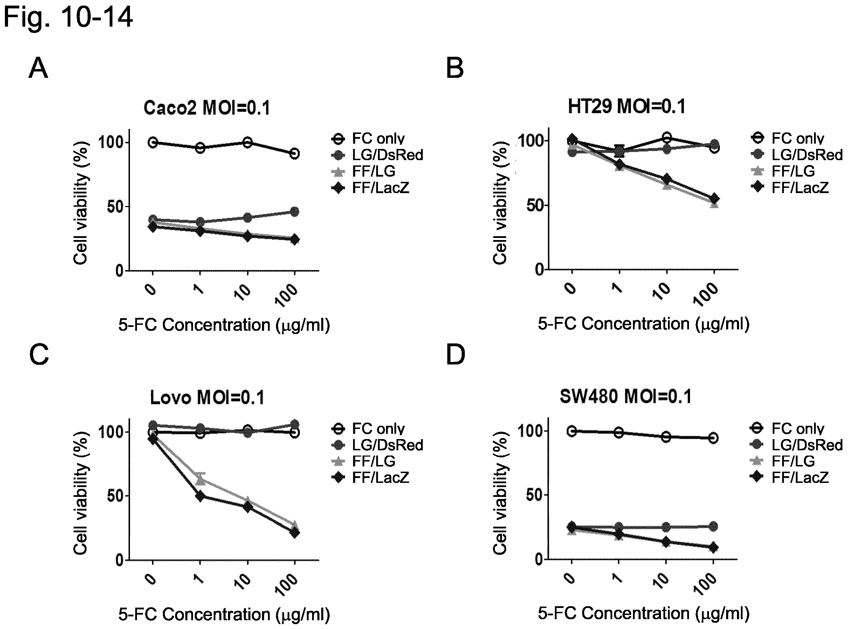

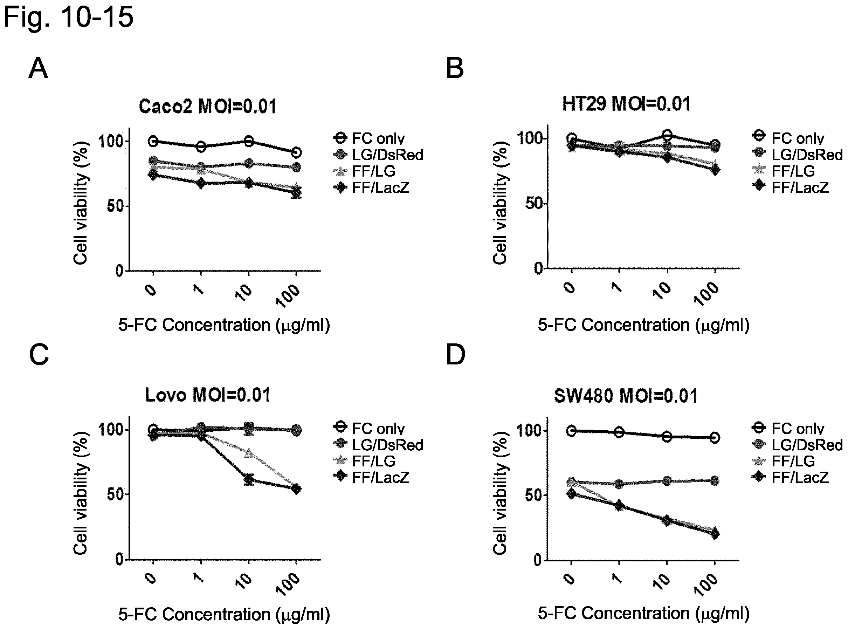

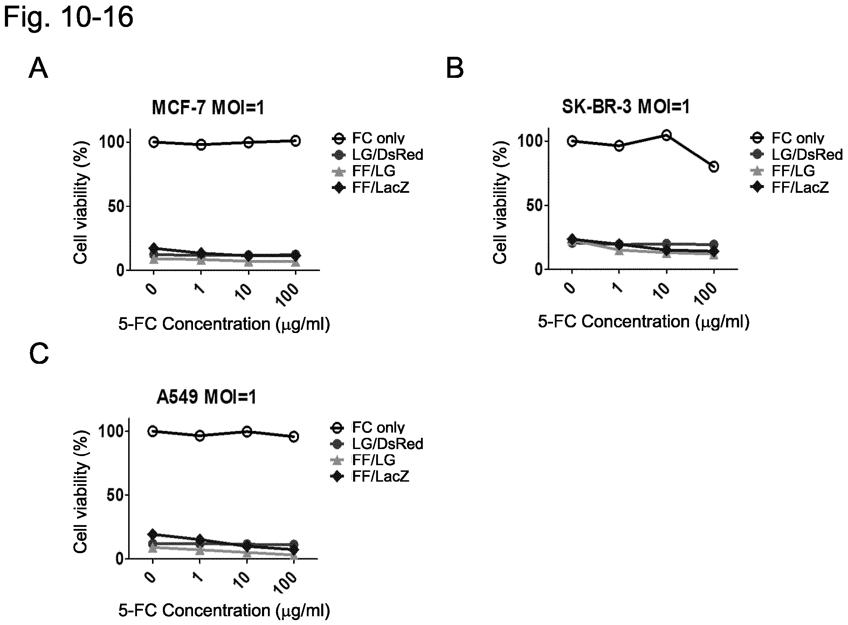

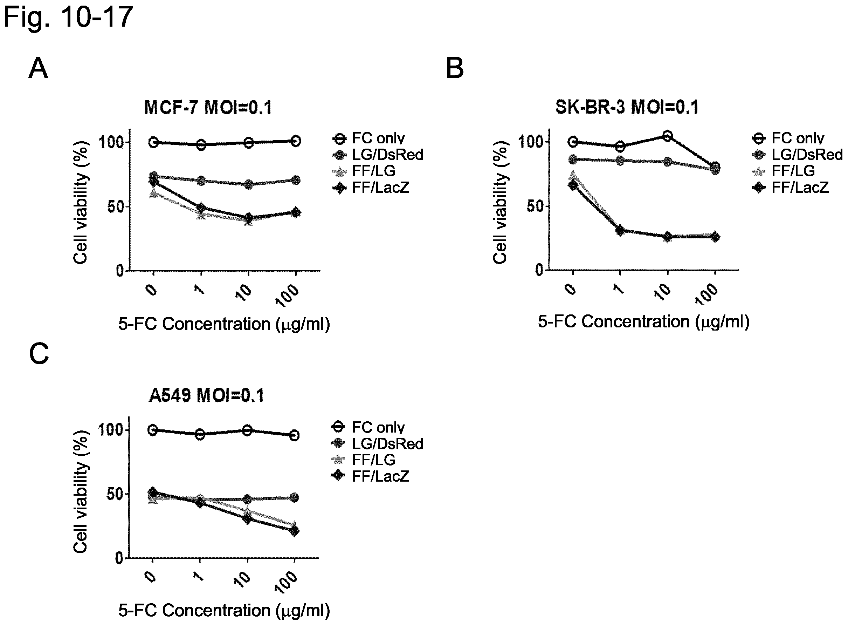

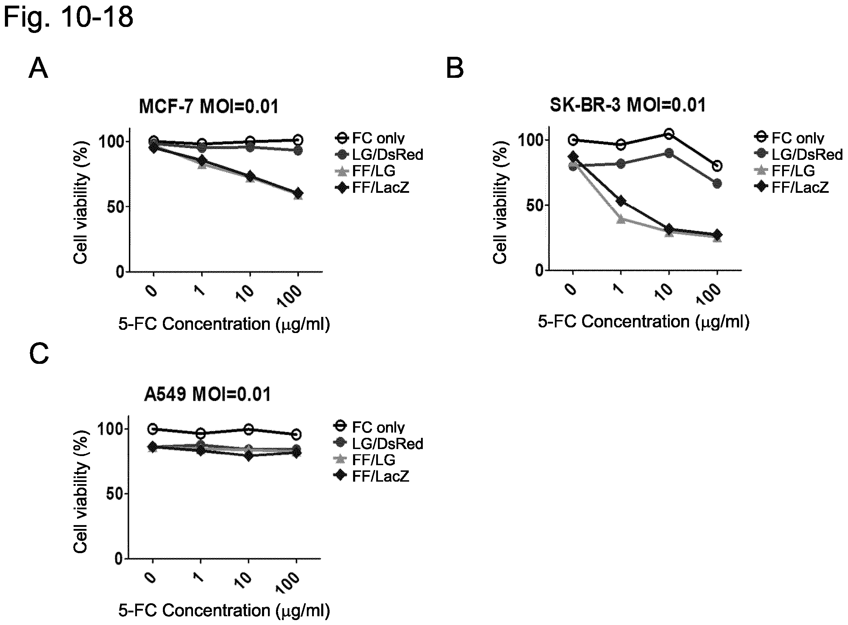

[Figure 10-1] Figures show the cell viabilities when human pancreatic cancer cells were infected at MOI = 1 with a mitogen-activated protein kinase (MAPK)-dependent recombinant vaccinia virus (MD-RVV) expressing cytosine deaminase (CD) gene and uracil phosphoribosyltransferase (UPRT) gene, which are the suicide genes as foreign genes, and subsequently 5-FC was added (A: AsPC1, B: AsPC1 CD44v9 High, C: BxPC3, D: MiaPaca2).

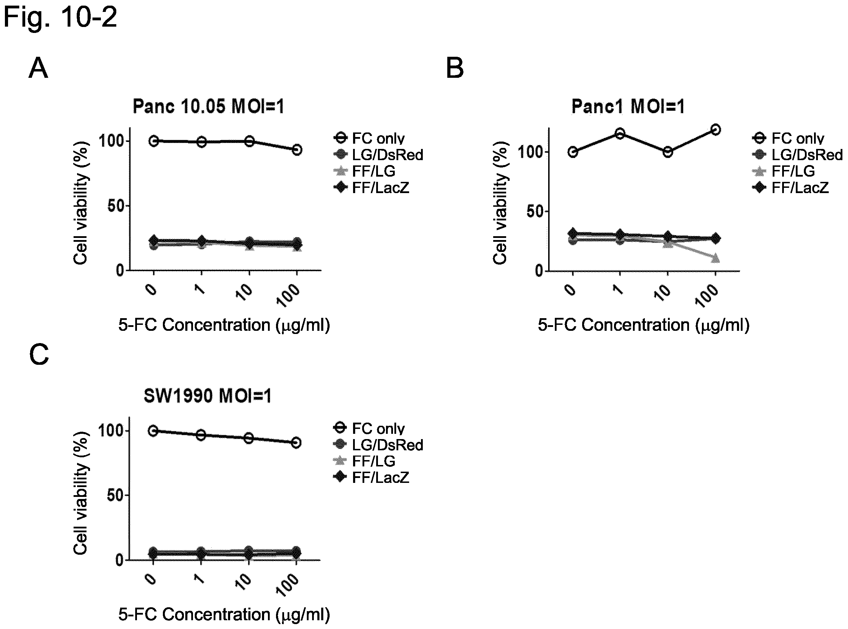

[Figure 10-2] Figures show the cell viabilities when human pancreatic cancer cells were infected at MOI = 1 with a mitogen-activated protein kinase (MAPK)-dependent recombinant vaccinia virus (MD-RVV) expressing cytosine deaminase (CD) gene and uracil phosphoribosyltransferase (UPRT) gene, which are the suicide genes as foreign genes, and subsequently 5-FC was add (A: Panc10.05, B: Panc1, C: SW1990).

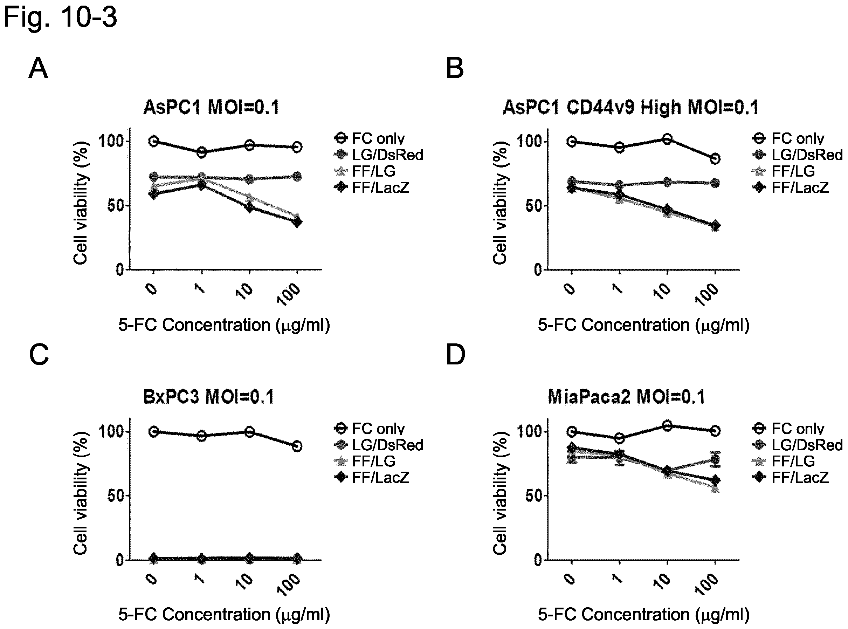

[Figure 10-3] Figures show the cell viabilities when human pancreatic cancer cells were infected at MOI = 0.1 with a mitogen-activated protein kinase (MAPK)-dependent recombinant vaccinia virus (MD-RVV) expressing cytosine deaminase (CD) gene and uracil phosphoribosyltransferase (UPRT) gene, which are the suicide genes as foreign genes, and subsequently 5-FC was add (A: AsPC1, B: AsPC1 CD44v9 High, C: BxPC3, D: MiaPaca2).

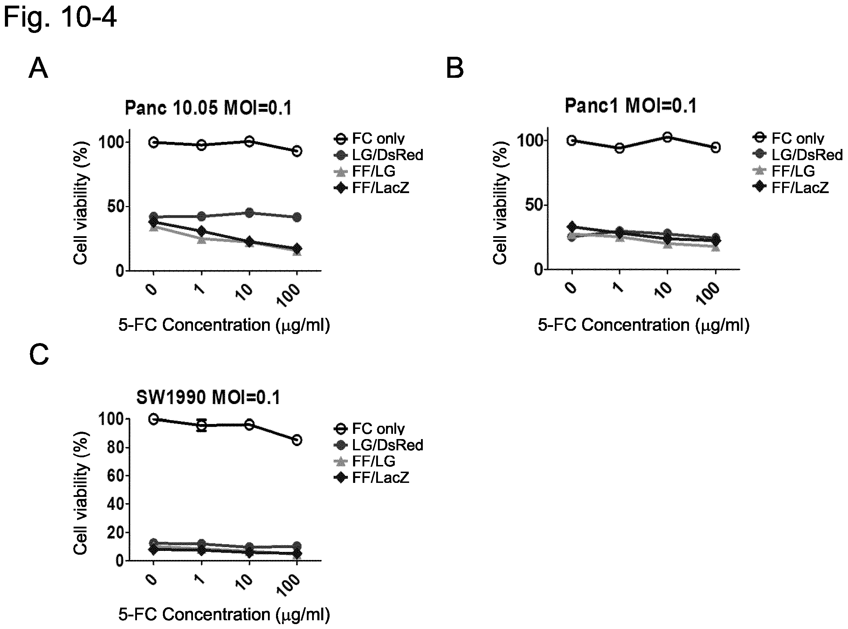

[Figure 10-4] Figures show the cell viabilities when human pancreatic cancer cells were infected at MOI = 0.1 with a mitogen-activated protein kinase (MAPK)-dependent recombinant vaccinia virus (MD-RVV) expressing cytosine deaminase (CD) gene and uracil phosphoribosyltransferase (UPRT) gene, which are the suicide genes as foreign genes, and subsequently 5-FC was add (A: Panc10.05, B: Panc1, C: SW1990).

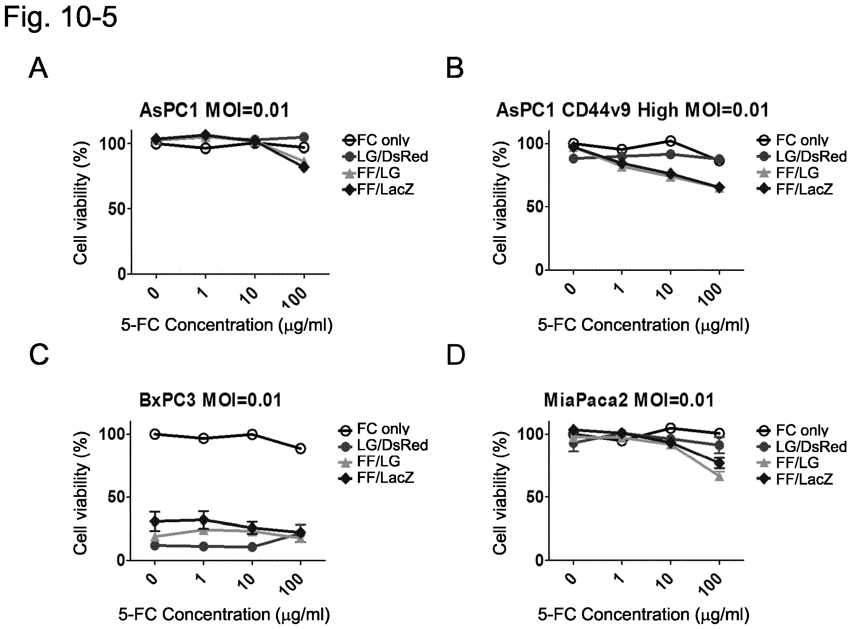

[Figure 10-5] Figures show the cell viabilities when human pancreatic cancer cells were infected at MOI = 0.01 with a mitogen-activated protein kinase (MAPK)-dependent recombinant vaccinia virus (MD-RVV) expressing cytosine deaminase (CD) gene and uracil phosphoribosyltransferase (UPRT) gene, which are the suicide genes as foreign genes, and subsequently 5-FC was add (A: AsPC1, B: AsPC1 CD44v9 High, C: BxPC3, D: MiaPaca2).

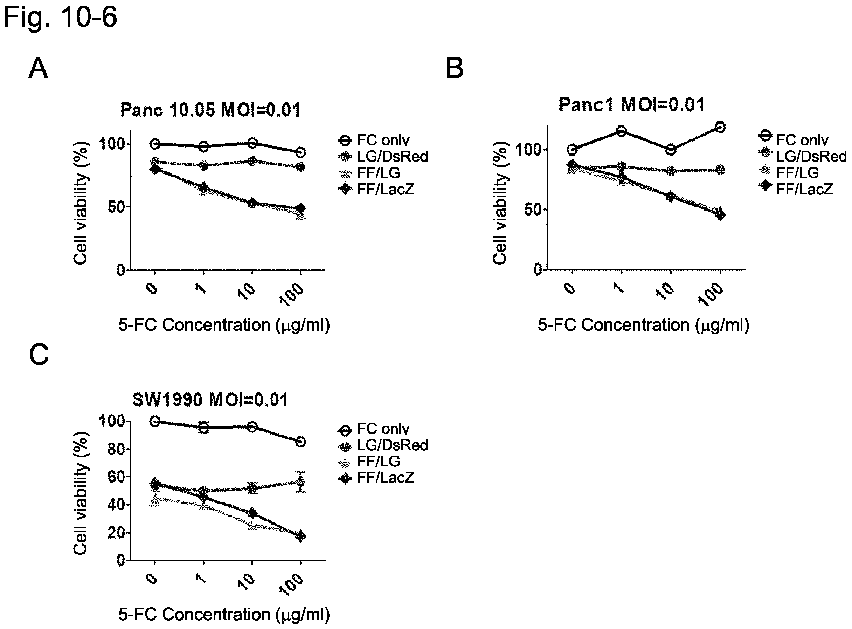

[Figure 10-6] Figures show the cell viabilities when human pancreatic cancer cells were infected at MOI = 0.01 with a mitogen-activated protein kinase (MAPK)-dependent recombinant vaccinia virus (MD-RVV) expressing cytosine deaminase (CD) gene and uracil phosphoribosyltransferase (UPRT) gene, which are the suicide genes as foreign genes, and subsequently 5-FC was add (A: Panc10.05, B: Panc1, C: SW1990).

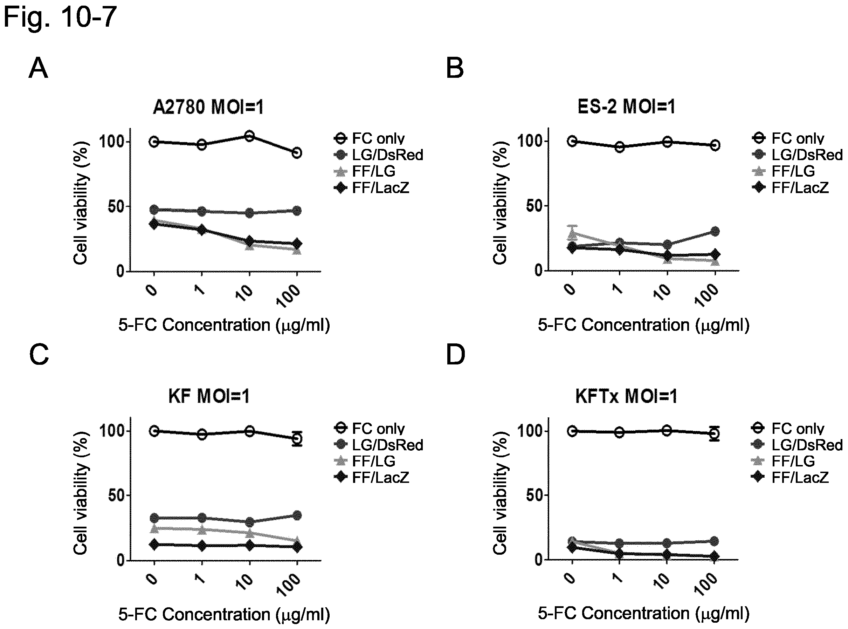

[Figure 10-7] Figures show the cell viabilities when human ovarian cancer cells were infected at MOI = 1 with a mitogen-activated protein kinase (MAPK)-dependent recombinant vaccinia virus (MD-RVV) expressing cytosine deaminase (CD) gene and uracil phosphoribosyltransferase (UPRT) gene, which are the suicide genes as foreign genes, and subsequently 5-FC was add (A: A2780, B: ES-2, C: KF, D: KFTx).

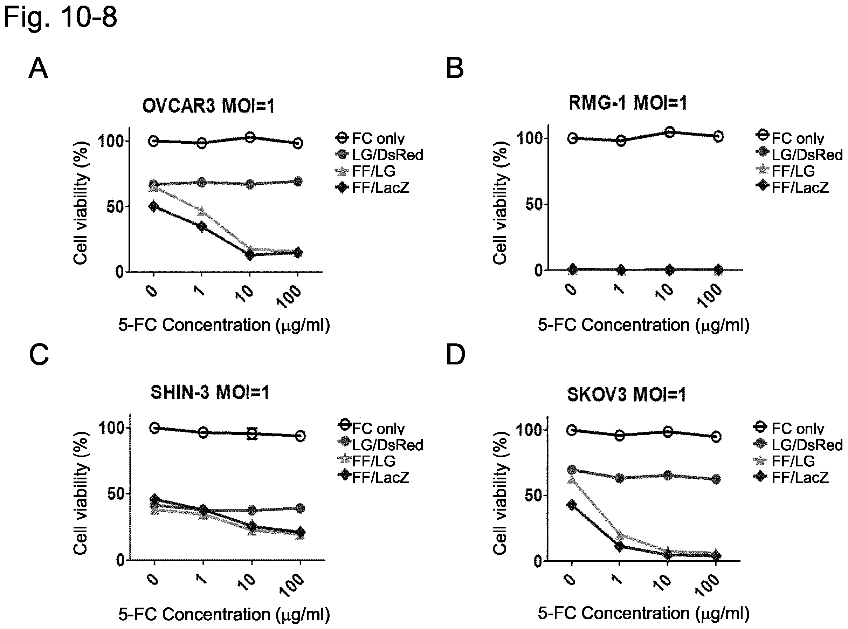

[Figure 10-8] Figures show the cell viabilities when human ovarian cancer cells were infected at MOI = 1 with a mitogen-activated protein kinase (MAPK)-dependent recombinant vaccinia virus (MD-RVV) expressing cytosine deaminase (CD) gene and uracil phosphoribosyltransferase (UPRT) gene, which are the suicide genes as foreign genes, and subsequently 5-FC was add (A: OVCAR3, B: RMG-1, C: SHIN-3, D: SKOV3).

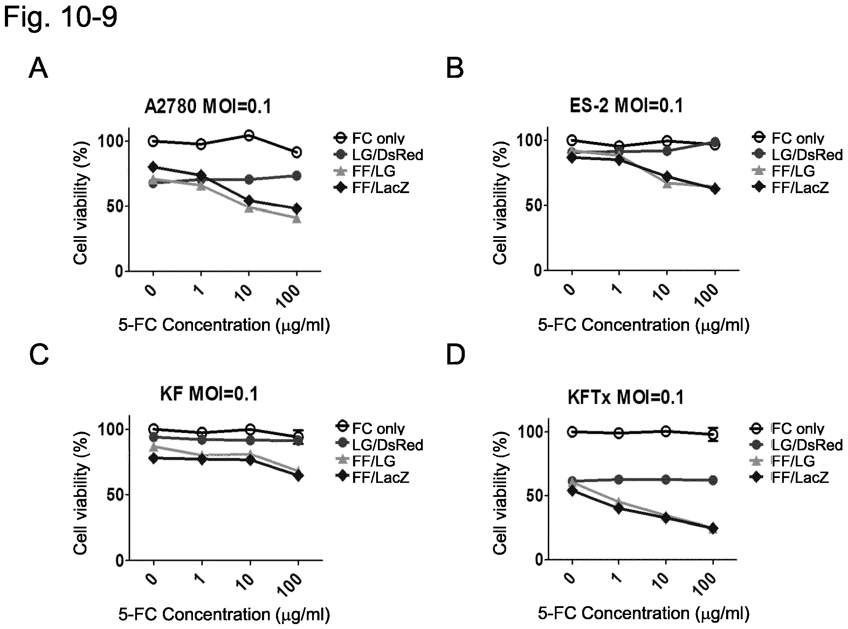

[Figure 10-9] Figures show the cell viabilities when human ovarian cancer cells were infected at MOI = 0.1 with a mitogen-activated protein kinase (MAPK)-dependent recombinant vaccinia virus (MD-RVV) expressing cytosine deaminase (CD) gene and uracil phosphoribosyltransferase (UPRT) gene, which are the suicide genes as foreign genes, and subsequently 5-FC was add (A: A2780, B: ES-2, C: KF, D: KFTx).
[Figure 10-10] Figures show the cell viabilities when human ovarian cancer cells were infected at MOI = 0.1 with a mitogen-activated protein kinase (MAPK)-dependent recombinant vaccinia virus (MD-RVV) expressing cytosine deaminase (CD) gene and uracil phosphoribosyltransferase (UPRT) gene, which are the suicide genes as foreign genes, and subsequently 5-FC was add (A: OVCAR3, B: RMG-1, C: SHIN-3, D: SKOV3).
[Figure 10-11] Figures show the cell viabilities when human ovarian cancer cells were infected at MOI = 0.01 with a mitogen-activated protein kinase (MAPK)-dependent recombinant vaccinia virus (MD-RVV) expressing cytosine deaminase (CD) gene and uracil phosphoribosyltransferase (UPRT) gene, which are the suicide genes as foreign genes, and subsequently 5-FC was add (A: A2780, B: ES-2, C: KF, D: KFTx).
[Figure 10-12] Figures show the cell viabilities when human ovarian cancer cells were infected at MOI = 0.01 with a mitogen-activated protein kinase (MAPK)-dependent recombinant vaccinia virus (MD-RVV) expressing cytosine deaminase (CD) gene and uracil phosphoribosyltransferase (UPRT) gene, which are the suicide genes as foreign genes, and subsequently 5-FC was add (A: OVCAR3, B: RMG-1, C: SHIN-3, D: SKOV3).
[Figure 10-13] Figures show the cell viabilities when human colon cancer cells were infected at MOI = 1 with a mitogen-activated protein kinase (MAPK)-dependent recombinant vaccinia virus (MD-RVV) expressing cytosine deaminase (CD) gene and uracil phosphoribosyltransferase (UPRT) gene, which are the suicide genes as foreign genes, and subsequently 5-FC was add (A: Caco2, B: HT29, C: Lovo, D: SW480).
[Figure 10-14] Figures show the cell viabilities when human colon cancer cells were infected at MOI = 0.1 with a mitogen-activated protein kinase (MAPK)-dependent recombinant vaccinia virus (MD-RVV) expressing cytosine deaminase (CD) gene and uracil phosphoribosyltransferase (UPRT) gene, which are the suicide genes as foreign genes, and subsequently 5-FC was add (A: Caco2, B: HT29, C: Lovo, D: SW480).
[Figure 10-15] Figures show the cell viabilities when human colon cancer cells were infected at MOI =0.01 with a mitogen-activated protein kinase (MAPK)-dependent recombinant vaccinia virus (MD-RVV) expressing cytosine deaminase (CD) gene and uracil phosphoribosyltransferase (UPRT) gene, which are the suicide genes as foreign genes, and subsequently 5-FC was add (A: Caco2, B: HT29, C: Lovo, D: SW480).
[Figure 10-16] Figures show the cell viabilities when human breast cancer cells or human lung cancer cells were infected at MOI = 1 with a mitogen-activated protein kinase (MAPK)-dependent recombinant vaccinia virus (MD-RVV) expressing cytosine deaminase (CD) gene and uracil phosphoribosyltransferase (UPRT) gene, which are the suicide genes as foreign genes, and subsequently 5-FC was add (A: MCF-7, B: SK-BR-3, C: A549).
[Figure 10-17] Figures show the cell viabilities when human breast cancer cells or human lung cancer cells were infected at MOI = 0.1 with a mitogen-activated protein kinase (MAPK)-dependent recombinant vaccinia virus (MD-RVV) expressing cytosine deaminase (CD) gene and uracil phosphoribosyltransferase (UPRT) gene, which are the suicide genes as foreign genes, and subsequently 5-FC was add (A: MCF-7, B: SK-BR-3, C: A549).
[Figure 10-18] Figures show the cell viabilities when human breast cancer cells or human lung cancer cells were infected at MOI = 0.01 with a mitogen-activated protein kinase (MAPK)-dependent recombinant vaccinia virus (MD-RVV) expressing cytosine deaminase (CD) gene and uracil phosphoribosyltransferase (UPRT) gene, which are the suicide genes as foreign genes, and subsequently 5-FC was add (A: MCF-7, B: SK-BR-3, C: A549).

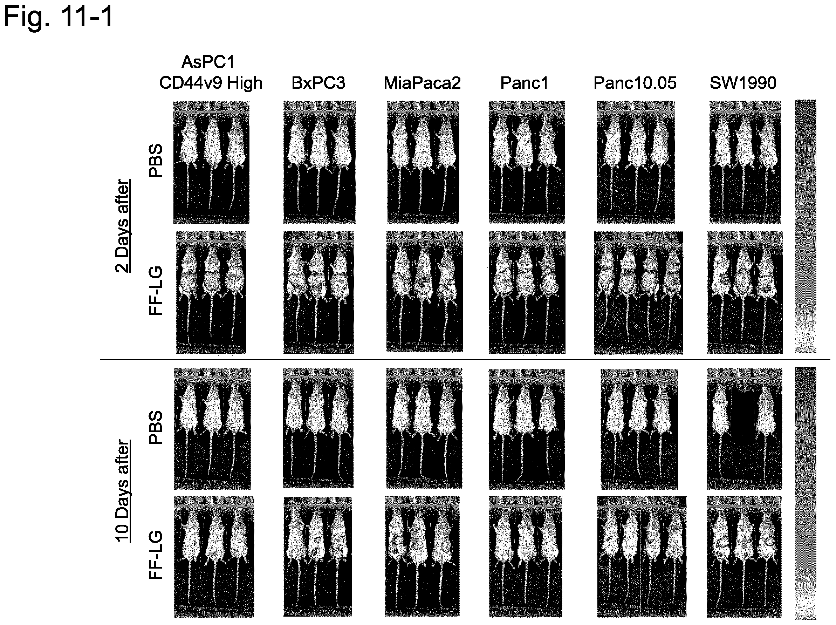

[Figure 11-1] Figures show the biodistribution of virus luciferase (Fluc) emission on day 2 and day 10 after the intraperitoneal administration of recombinant vaccinia virus, LC16mO VGF-EL-Fcy::Fur/O1L-EL-LucGFP, at 1 x 10⁶ PFU/mouse to peritoneally tumor-disseminated SCID mouse models in which 6 types of human pancreatic cancer cells (AsPC1 CD44v9 High, BxPC3, MiaPaca2, Panc10.05, Panc1, and SW1990) constantly expressing *Renilla*-derived luciferase (Rluc) were intraperitoneally transplanted.

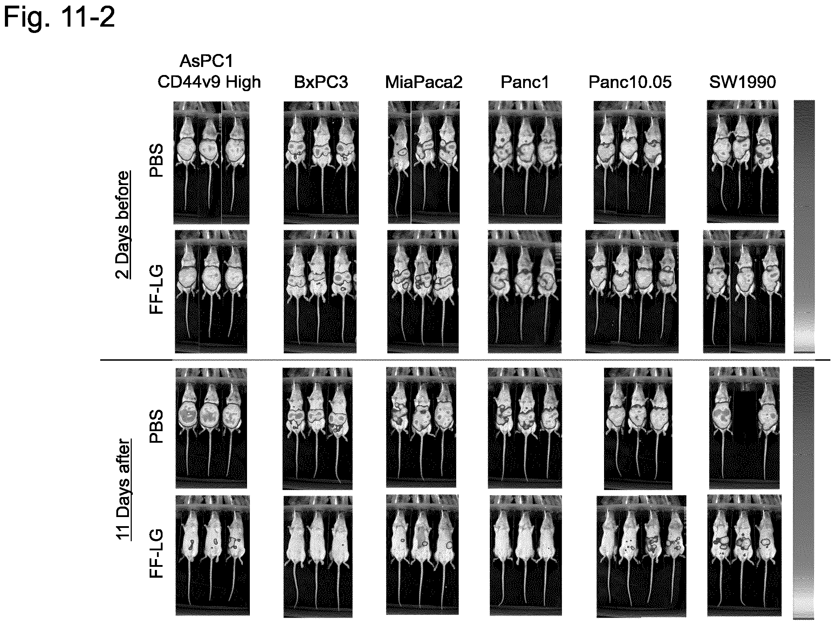

[Figure 11-2] Figures show the biodistribution of tumor Rluc emission 2 days before and on day 11 after the intraperitoneal administration of recombinant vaccinia virus, LC16mO VGF-EL-Fcy::Fur/O1L-EL-LucGFP, at 1 x 10⁶ PFU/mouse to peritoneally tumor-disseminated SCID mouse models in which 6 types of human pancreatic cancer cells (AsPC1 CD44v9 High, BxPC3, MiaPaca2, Panc10.05, Panc 1 and SW1990) constantly expressing *Renilla*-derived luciferase (Rluc) were intraperitoneally transplanted.

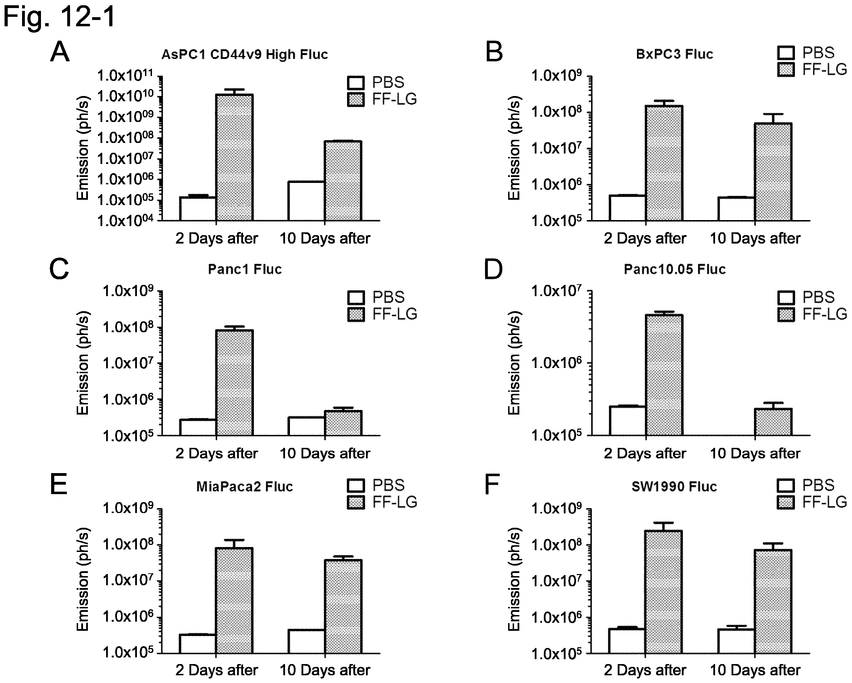

[Figure 12-1] Figures show the quantified results on the virus Fluc emission in the body of mice shown in Figure 11-1 (A: AsPC1 CD44v9 High, B: BxPC3, C: Panc1, D: Panc10.05, E: MiaPaca2, and F: SW1990).

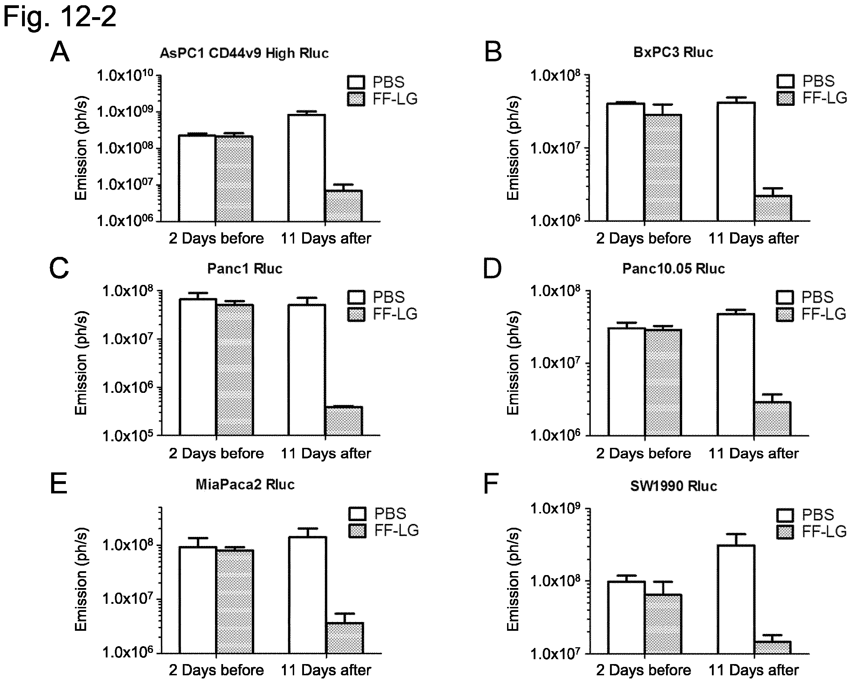

[Figure 12-2] Figures show the quantified results on the tumor Rluc emission in the body of mice shown in Figure 11-1 (A: AsPC1 CD44v9 High, B: BxPC3, C: Panc1, D: Panc10.05. E: MiaPaca2, and F: SW1990).

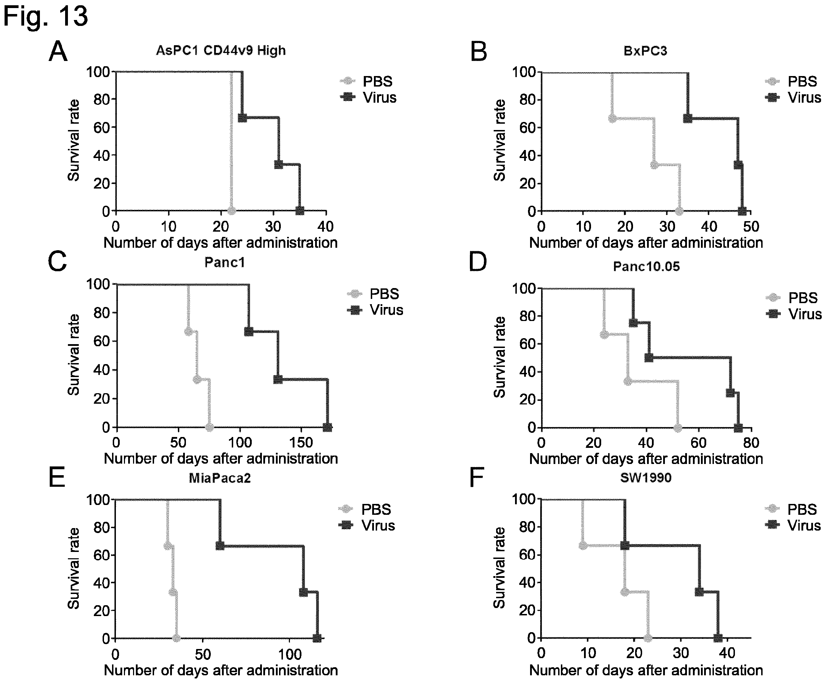

[Figure 13] Figures show survival rates after virus (LC16mO VGF-EL-Fcy::Fur/O1L-EL-LucGFP) was administered to peritoneally human pancreatic cancer-disseminated models (A: AsPC1 CD44v9 High, B: BxPC3, C: Panc1, D: Panc10.05, E: MiaPaca2, and F: SW1990).

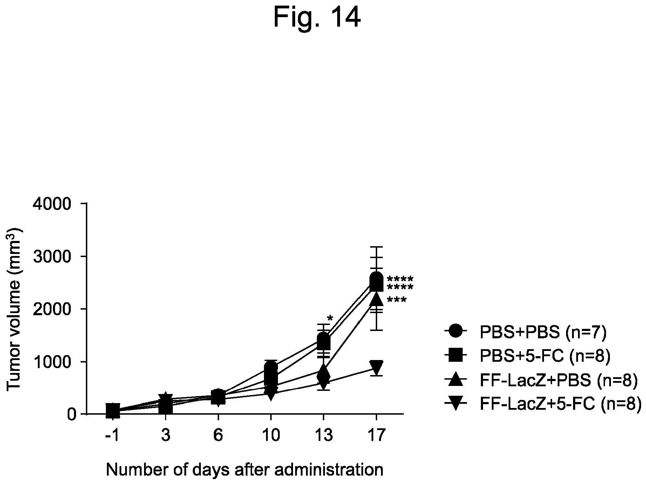

[Figure 14] Figure shows changes in the tumor size after LC16mO VGF-EL-Fcy::Fur/O1L-EL-LacZ at 1x10⁸ PFU/mouse into the tumor and further 12.5 mg of 5-FC intraperitoneally were alternately administered 6 times each to subcutaneously tumor-transplanted B6 mouse models using mouse colon cancer cell MC38.

### Description of Embodiments

Hereinafter, the present invention is described in detail.

The present invention relates to a method for introducing a foreign gene to a vaccinia virus and a vaccinia virus, having suicide genes, cytosine deaminase (CD) gene and uracil phosphoribosyltransferase (UPRT) gene, inserted into one of the vaccinia virus growth factor (VGF) and the OIL gene, and a marker gene inserted into the other of the vaccinia virus growth factor (VGF) and the OIL gene, being deprived of functions of the vaccinia virus growth factor (VGF) and the OIL gene, not growing in normal cells and growing specifically in cancer cells, and having oncolytic effect damaging specifically cancer cells; and further killing cancer cells by the action of the suicide genes as well as the vaccinia virus according to the present invention for use in treating cancer.

For the vaccinia virus expressing a foreign gene of the present invention, a marker gene is first inserted into an endogenous gene or an untranslated region of a vaccinia virus.

Subsequently, the inserted marker gene is replaced with a foreign gene of interest to be inserted into the vaccinia virus.

At the time, the replacement of the marker gene with the foreign gene of interest is confirmed using the signal caused by the expression product of the marker gene as an indicator. More specifically, when the marker gene is replaced with the foreign gene, the signal from the expression product of the marker gene is lost, whereby the replacement of the marker gene with the foreign gene can be determined using the loss of the signal as the indicator. For example, in the case where the marker gene is a fluorescent protein gene, the replacement of the marker gene with the foreign gene can be determined using the existence of the fluorescence emitted by the fluorescent protein as the indicator.

The vaccinia virus expressing a foreign gene of the present invention can also be prepared by using a vaccinia virus having a marker gene inserted into an endogenous gene or an untranslated region originally present therein. The vaccinia virus having a marker gene inserted into an endogenous gene originally present therein refers to, for example, a vaccinia virus having a marker gene inserted into an endogenous gene for the purpose of causing loss of the function of a specific endogenous gene.

The endogenous gene into which a marker gene is first inserted is not limited, but a gene which is not required for the life cycle of the vaccinia virus is preferable. Alternatively, a marker gene can be inserted into a gene for which even when loss of the function occurs by insertion of a marker gene, the loss can be compensated in cancer cells by abundant enzymes of cancer cells, but the loss of the function cannot be compensated in normal cells and the like.

Examples of the endogenous gene include vaccinia virus growth factor (VGF) gene (U.S. Patent Application No. 2003/0031681 Description); OIL gene; hemagglutinin (HA) gene; thymidine kinase (TK) gene; F fragment; F3 gene; bleeding region or A-type inclusion body region (US Patent No. 6,596,279 Description); Hind III F, F13L or Hind III M region (US Patent No. 6,548,068 Description); A33R, A34R or A36R gene (Katz et al., J. Virology 77: 12266-12275 (2003)); SalF7L gene (Moore et al., EMBO J. 1992 11:1973-1980); NIL gene (Kotwal et al., Virology 1989 171:579-58); M1 gene (Child et al., Virology. 1990 174:625-629); HR, HindIII-MK, HindIII-MKF, HindIII-CNM, RR or BamF region (Lee et al., J Virol. 1992 66:2617-2630); and C21L gene (Isaacs et al., Proc Natl Acad Sci U S A. 1992 89:628-632). Of these genes, VGF gene, OIL gene, TK gene, HA gene, and F fragment are preferable.

A marker gene may be inserted into one or more, for example, 1 or 2, or 1 to 3, of these endogenous genes.

For example, lacking the function of the TK by inserting a marker gene into the TK gene of the vaccinia virus means that the TK gene is not expressed or, even if expressed, the expressed protein does not retain the normal TK function. When the function of the TK gene is lost, the growth ability of the vaccinia virus in normal cells declines. On the other hand, the growth ability in cancer cells does not decline because there are abundant enzymes compensating this gene function present in cancer cells. The declined growth ability in normal cells means that the pathogenicity against normal cells declines, more specifically, the safety is improved when applied to a living body.

Further, lacking the functions of the VGF and OIL genes by inserting a marker gene into VGF and OIL genes of the vaccinia virus means that the gene encoding VGF and the gene encoding OIL are not expressed or, even if expressed, the expressed protein does not retain the normal functions of VGF and OIL. As an example of the endogenous gene, the gene sequences of VGF and OIL of the vaccinia virus are presented respectively as set forth in SEQ ID NOS: 10 and 11. When the vaccinia virus being deprived of the functions of VGF and OIL infects normal cells, ERK is not activated in normal cells and thus the cell growth is not promoted, resulting in the notable decline of vaccinia virus growth. On the other hand, the Ras/Raf/MEK/ERK metabolic pathway abnormally activated in cancer cells compensates the activation function of ERK by VGF and OIL of the vaccinia virus, whereby the vaccinia virus can grow. As a result, the vaccinia virus grows specifically in cancer cells and damages the cancer cells causing destruction. More specifically, the mitogen-activated protein kinase-dependent recombinant vaccinia virus of the present invention has the cancer cell-specific oncolytic effect.

The untranslated region refers to regions which are present on both sides of the coding region of mRNA and not translated to a protein, and includes the 5' untranslated region and the 3' untranslated region. The untranslated region of the present invention includes both of the untranslated regions.

The marker gene to be inserted into the endogenous gene or the untranslated region described above is also called a reporter gene and examples include fluorescent protein genes such as luciferase (LUC) gene and green fluorescent protein (GFP), fluorescent protein genes such as red fluorescent protein (DsRed), β glucuronidase (GUS) gene, chloramphenicol acetyltransferase (CAT) gene, and β-galactosidase (LacZ) gene.

The insertion of a marker gene into the endogenous gene or the untranslated region may be carried out by, for example, homologous recombination. Homologous recombination is a phenomenon in which two DNA molecules are mutually recombined via the same nucleotide sequence in a cell and is the method often used for recombining viruses having an enormous genetic DNA such as the vaccinia virus. First, a plasmid to which a marker gene is connected (this is called "transfer vector") is constructed in the form of splitting at the center of the DNA sequence of the endogenous gene site or the untranslated region of the vaccinia virus to be targeted and introduced into cells infected with the vaccinia virus, whereby the recombination is caused between the virus DNA which becomes naked in the process of the virus replication and the same sequence part on the transfer vector, thereby integrating the interposed marker gene into the virus genome. For the cell to be used herein, a vaccinia virus-infectable cell such as CV1 cell, RK13 cell, BSC-1 cell, HTK-143 cell, Hep2 cell, MDCK cell, Vero cell, HeLa cell, COS cell, BHK-21 cell, or primary rabbit kidney cell can be used. Additionally, the introduction of the vector to a cell may be carried out by a well-known method such as calcium phosphate method, cationic liposome method, or electroporation method.

For example, Figure 1 shows the structure of a plasmid (shuttle vector) for easily and quickly inserting a marker gene expression unit into the VGF gene or the OIL gene, which is the endogenous gene of the vaccinia virus. The present disclosure also encompasses the plasmid for inserting a marker gene expression unit into the endogenous gene of the vaccinia virus.

Further, when the marker gene is introduced, it is desirable to operably link a suitable promoter upstream of the marker gene. The promoter is not limited but PSFJ1-10, PSFJ2-16, p7.5K promoter, p11K promoter, T7.10 promoter, CPX promoter, HF promoter, H6 promoter, T7 hybrid promoter, or the like can be used. The method for introducing the marker gene to the vaccinia virus vector of the present invention can be carried out by a well-known method for constructing a recombinant vaccinia virus vector by following, for example, the description in Experimental Medicine Supplement, The protocol series, Gene transfer & Experimental method of analyzing expression, edited by Izumi Saito et al., YODOSHA CO., LTD. (published September 1, 1997) or D. M. Glover et al. edition, supervised by Ikunoshin Kato, DNA Cloning 4 -Mammalian Systems- (2nd edition) TaKaRa, EMBO Journal, 1987, vol. 6, p. 3379-3384.

The vaccinia viruses having marker genes inserted in the endogenous genes originally found therein are described, for example, in International Publication No. WO2011/125469 and International Publication No. WO2015/076422, and can be prepared by following the methods described in these publications. International Publication No. WO2011/125469 describes the vaccinia virus having marker genes inserted into the endogenous genes, TK gene, HA gene, and so on and having the functions of TK and HA lost. Additionally, International Publication No. WO2015/076422 describes the vaccinia virus having marker genes inserted into the VGF (vaccinia virus growth factor) gene and the OIL gene and having the functions of VGF (vaccinia virus growth factor) and OIL lost. This vaccinia virus is called the mitogen-activated protein kinase (MAPK)-dependent recombinant vaccinia virus (MD-RVV).

The vaccinia virus strain for producing the vaccinia virus having a marker gene inserted into the endogenous gene originally found therein is not limited, and examples include stains such as Lister strain, LC16 strain, LC16mO strain, and LC16m8 strain established from Lister strain (So Hashizume, Clinical Virology, vol.3, No.3, 269, 1975, etc.), and NYBH strain, Wyeth strain, Copenhagen strain, WR strain, and MVA strain. LC16mO strain is the strain generated from Lister strain via LC16 strain, and LC16m8 strain is further generated from LC16mO strain and attenuated strain because the B5R gene encoding a viral membrane protein had a frameshift mutation and thereby the protein was not expressed or did not function (PROTEIN, NUCLEIC ACID AND ENZYME, Vol.48 No. 12 (2003), p. 1693-1700).

The vaccinia virus having a marker gene inserted into the endogenous gene originally found therein used in the present invention is preferably attenuated and not pathogenic. Examples of the attenuated strain include strains having the B5R gene partially or completely deleted. The B5R gene encodes the protein present in the envelope of vaccinia virus and the B5R gene product is involved with the infection and growth of the virus. The B5R gene product is present on the infected cell surface and in the envelope of the virus, works to increase the efficiency when the virus infects and propagates in adjacent cells or other parts of the host body, and is also involved with the plaque size and the range of host. When the virus having the B5R gene deleted is caused to infect animal cells, the plaque size reduces and the pock size also reduces. Additionally, the growth ability on the skin declines, and the pathogenicity to the skin declines. For the vaccinia virus having the B5R gene partially or completely deleted, the B5R gene product fails to function normally, has the reduced skin growth ability, and does not cause adverse reaction when administered to human. Examples of the attenuated strain with the B5R gene deleted include m8Δ strain established by completely deleting the B5R gene, for example, from the above LC16m8 strain (also called LC16m8Δ strain). Additionally, mOΔ strain established by completely deleting the B5R gene from LC16mO strain (also called LCmOΔ strain) can also be used. These attenuated vaccinia virus strains having the B5R gene partially or completely deleted are described in International Publication No. WO2005/054451 and can be obtained by following the description therein. Whether or not the vaccinia virus has the B5R gene partially or completely deleted and the function of the B5R protein is lost can be determined, for example, by the size of plaque and the size of pock formed when infected with RK13 cells, the virus growth properties in vero cells, and the pathogenicity to the skin in rabbit as indicators. Alternatively, the gene sequence of vaccinia virus may also be investigated.

The vaccinia virus expressing a foreign gene of the present invention, when used for a cancer therapy, is designed in such a way as to express the B5R gene in cancer cells and cause the damage of the cancer cells by the action of the B5R protein. In this case, it is desirable that the vaccinia virus to be used express the complete B5R gene. When the vaccinia virus which does not have the B5R gene and is attenuated and established the safety as described above is used, a new complete B5R gene is newly introduced into the vaccinia virus having the B5R gene deleted. When the vaccinia virus having B5R gene partially or completely deleted is used, a B5R gene is inserted into such a vaccinia virus genome and may be used as a material for the vaccinia virus production of the present invention. The insertion of the B5R gene into the vaccinia virus may be carried out by any method and may be carried out by, for example, a known homologous recombination method. Further, the position at which the B5R gene is inserted in this case may be between the B4R gene and the B6R gene where the B5R gene was originally present, or at any site in the genome of the vaccinia virus. Further, the B5R gene may be constructed as a DNA construct in advance and introduced into the vaccinia virus.

Examples of the foreign gene (foreign DNA or foreign polynucleotide) with which the marker gene is replaced include therapeutic genes encoding products having the cytotoxicity and immunostimulatory effect, and further DNA encoding a protein antigen of a cancer, a virus, a bacterium, a protozoan, or the like. Further examples include suicide genes such as cytosine deaminase (CD) gene, uracil phosphoribosyltransferase (UPRT) gene, herpes simplex virus thymidine kinase (HSV-tk) gene, guanine phosphoribosyltransferase (gpt) gene, and nitroreductase gene.

All of the marker genes inserted into the endogenous gene may be replaced with foreign genes, or one or more of the marker genes, for example, 1 or 2, or 1 to 3 marker genes, may be replaced with foreign genes.

The therapeutic gene is the gene used for treating specific diseases such cancers and infectious diseases and examples include tumor suppressor genes such as p53 and Rb; genes encoding a physiologically active substance such as interleukin 1 (IL-1), IL-2, IL-3, IL-4, IL-5, IL-6, IL-7, IL-8, IL-9, IL-10, IL-11, IL-12, IL-13, IL-14, IL-15, α-interferon, β-interferon, γ-interferon, angiostatin, thrombospondin, endostatin, METH-1, METH-2, GM-CSF, G-CSF, M-CSF, or a tumor necrosis factor; and genes encoding sodium iodide symporter (NIS). The gene encoding sodium iodide symporter (NIS) may be used in combination with a radioactive iodine, thereby enabling the delivery of a radioactive isotope to tumor tissues. When the vaccinia virus expressing a foreign gene of the present invention is used for a cancer therapy, a therapeutic gene against a cancer, together with the oncolysis provided by the vaccinia virus, can demonstrate cancer therapeutic effects.

Further, when DNA encoding an antigen of a virus, a bacterium, a protozoan, a cancer, or the like is introduced as a foreign gene (foreign DNA), the vaccinia virus vector into which the foreign gene is introduced can be used as the vaccine against various viruses, bacteria, protozoans, and cancers. For example, an infection protective antigen (neutralizing antigen) such as human immunodeficiency virus, hepatitis virus, herpes virus, micobacterium, malaria plasmodium, severe acute respiratory syndrome (SARS) virus, or a gene encoding a cancer antigen may be introduced.

These foreign genes can be introduced by, for example, using the technique of homologous recombination. The homologous recombination may be carried out by the method described above. For example, a plasmid (transfer vector) into which a foreign gene to be introduced is linked to a DNA sequence at an introduction site is prepared, which may be introduced into a cell infected with the vaccinia virus. The introduction region for the foreign gene is preferably a gene which is not required for the life cycle of the vaccinia virus. Alternatively, in cancer cells, the foreign gene may also be inserted, for example, into a gene for which, even when the function is lost by the insertion of the gene, the lost function can be compensated by abundant enzymes found in cancer cells but not compensated in normal cells.

Further, when the foreign gene is introduced, it is desirable to operably link a suitable promoter upstream of the foreign gene. The promoter is not limited but PSFJ1-10, PSFJ2-16, p7.5K promoter, p11K promoter, T7.10 promoter, CPX promoter, HF promoter, H6 promoter, T7 hybrid promoter, or the like, described above can be used. The method for introducing the foreign gene to the vaccinia virus vector of the present invention can be carried out by a well-known method for constructing a recombinant vaccinia virus vector by following, for example, the description in Experimental Medicine Supplement, The protocol series, Gene transfer & Experimental method of analyzing expression, edited by Izumi Saito et al., YODOSHA CO., LTD. (published September 1, 1997) or D. M. Glover et al. edition, supervised by Ikunoshin Kato, DNA Cloning 4 -Mammalian Systems- (2nd edition) TaKaRa, EMBO Journal, 1987, vol. 6, p. 3379-3384, and so on.

When the vaccinia virus is used for a cancer therapy, the cancer to be targeted is not limited and various cancer types, when classified by the cancer-causing organ, are targeted, such as lung cancer, pancreatic cancer, ovarian cancer, skin cancer, stomach cancer, liver cancer, colorectal cancer, colon cancer, anal·rectal cancer, esophagus cancer, uterine cancer, breast cancer, bladder cancer, prostate cancer, esophagus cancer, brain·nerve tumors, lymphoma, leukemia, bone·bone sarcoma, leiomyoma, and rhabdomyoma.

The pharmaceutical composition for cancer therapy comprising the vaccinia virus of the present invention comprises a pharmaceutically effective amount of the vaccinia virus of the present invention as an effective ingredient and may be in the form of a sterile aqueous or nonaqueous solution, suspension, or emulsion. Further, the composition may comprise a pharmaceutically acceptable diluent, aid, or carrier such as a salt, a buffer agent, and an adjuvant. The administration may be carried out via various parenteral routes such as subcutaneous route, intravenous route, intradermal route, intramuscular route, intraperitoneal route, intranasal route, or transdermal route. Additionally, local administration may be carried out to a cancer site. The effective dose can be suitably determined by the age, sex, health conditions, and body weight of a subject. For example, the dose is, but not limited thereto, about 10² to 10¹⁰ plaque-forming unit (PFU) per administration to a human adult.

For example, when the CD gene, which is a suicide gene, is used as the foreign gene, 5-fluorocytosine (5-FC) is administered to a subject together with the vaccinia virus capable of expressing the CD gene. Cytosine deaminase (CD), which is an expression product of the CD gene, converts 5-FC to 5-fluorouracil (5-FU), which is a cytotoxic anticancer agent, whereby the anticancer effect is demonstrated. Further, when the UPRT gene, which is a suicide gene, is used as the foreign gene, 5-fluorouracil (5-FU) is administered to a subject together with the vaccinia virus capable of expressing the UPRT gene. Uracil phosphoribosyltransferase (UPRT), which is an expression product of the UPRT gene, enhances the anticancer effect of 5-FU.

Both CD gene and UPRT gene may be used as the foreign genes, and in this case, 5-FC is administered to a subject together with the vaccinia virus capable of expressing the CD gene and the UPRT gene. 5-FC is converted to 5-FU by cytosine deaminase (CD), which is an expression product of the CD gene, and the anticancer agent effect is further enhanced by uracil phosphoribosyltransferase (UPRT), which is an expression product of the UPRT gene.

Further, when the herpes simplex virus thymidine kinase (HSV-tk) gene, which is a suicide gene, is used as the foreign gene, ganciclovir (GCV), aciclovir, penciclovir or the like is administered to a subject together with the vaccinia virus. GCV itself does not have a drug activity but is monophosphorylated by HS-tk and further converted to triphosphate in the cell. The triphosphorylated GCV attacks cancer cells infected with the vaccinia virus to kill the cancer cells.

Furthermore, when the guanine phosphoribosyltransferase (gpt) gene, which is a suicide gene, is used as the foreign gene, 6-thioxanthine or 6-thioguanine may be administered, and when the nitroreductase gene is used, CB1954 may be administered.

In the case where the vaccinia virus grows in normal cells and causes an adverse reaction, if a suicide gene is expressed in the vaccinia virus, the growth of the virus can be stopped by the action of the suicide gene and thereby the adverse reaction is suppressed.

The present invention pertains to vaccinia viruses comprising a suicide gene as a foreign gene and expressing the suicide gene. The suicide genes are inserted into one of the vaccinia virus growth factor (VGF) gene and the OIL gene, wherein a marker gene is inserted into the other of the vaccinia virus growth factor (VGF) gene and the OIL gene, and such a virus is deprived of functions of the vaccinia virus growth factor (VGF) gene and the OIL gene, and has oncolytic effect of not growing in normal cells but growing specifically in cancer cells, and destroying specifically the cancer cells, and further can kill the cancer cells by an action of the suicide gene.

Further, as illustrated in Figure 3, a suicide gene may be inserted into the vaccinia virus growth factor (VGF) gene or the OIL gene, and a marker gene is inserted into the vaccinia virus growth factor (VGF) gene or the OIL gene into which the suicide gene is not inserted. Examples of the marker gene include the above β-galactosidase (LacZ) gene.

Examples of the suicide gene include cytosine deaminase (CD) gene, uracil phosphoribosyltransferase (UPRT) gene, herpes simplex virus thymidine kinase (HSV-tk) gene, guanine phosphoribosyltransferase (gpt) gene and nitroreductase gene, and a fusion gene of a plurality of suicide genes may be inserted and examples include a fusion gene of the cytosine deaminase (CD) gene and the uracil phosphoribosyltransferase (UPRT) gene. In the vaccinia virus according to the present invention, the suicide genes are the cytosine deaminase (CD) gene and the uracil phosphoribosyltransferase (UPRT) gene.

Examples of the vaccinia virus of the present invention include the vaccinia virus (VGF-EL-Fcy::Fur/O1L-EL-LacZ) wherein a fusion gene of the cytosine deaminase (CD) gene and the uracil phosphoribosyltransferase (UPRT) gene is inserted into the vaccinia virus growth factor (VGF) gene and further the β-galactosidase (LacZ) gene is inserted into the OIL gene.

The present disclosure encompasses a pharmaceutical composition for cancer therapy, more specifically, an anticancer agent, comprising such a vaccinia virus as an effective ingredient. The pharmaceutical composition can be used in combination with 5-fluorocytosine (5-FC) or 5-fluorouracil (5-FU). For example, when only the cytosine deaminase (CD) gene is, or the cytosine deaminase (CD) gene and the uracil phosphoribosyltransferase (UPRT) gene are, or the fusion gene thereof is inserted into the vaccinia virus, the composition may be used in combination with 5-fluorocytosine (5-FC), and when only the uracil phosphoribosyltransferase (UPRT) gene is inserted into the vaccinia virus, the composition may be used in combination with 5-fluorouracil (5-FU).

The present disclosure encompasses a combined formulation or a combination kit of the above vaccinia virus and 5-fluorocytosine (5-FC) or 5-fluorouracil (5-FU). Further, the present disclosure encompasses the pharmaceutical composition comprising the vaccinia virus as the effective ingredient and used in combination with 5-fluorocytosine (5-FC) or 5-fluorouracil (5-FU). The vaccinia virus and 5-fluorocytosine (5-FC) or 5-fluorouracil (5-FU) may be administered simultaneously or may be administered separately. Further, these ingredients may be sequentially administered, and the administration order may be the vaccinia virus being first or 5-fluorocytosine (5-FC) or 5-fluorouracil (5-FU) being first.

### Examples

The present invention is described specifically in reference to the following Examples but is not limited to these Examples.

### Example 1 Platform for generating gene recombinant vaccinia

For easily and quickly inserting a foreign gene expression unit to the VGF gene or the OIL gene, a BFP gene region was first amplified using two primers (SEQ ID NO: 1 and SEQ ID NO: 2) using the DNA of pTagBFP-N (FP172, Evrogen) as a template. Each of the PCR products was cleaved with the restriction enzymes SfiI and EcoRI and cloned into the same restriction enzyme sites of the pTK-SP-LG vector (International Publication No. WO2015/076422) to construct pTK-SP-BFP wherein BFP is linked under the synthetic vaccinia virus promoter (Hammond JM. et al., Journal of Virological Methods. 1997; 66(1): 135-138). Subsequently, the pTK-SP-BFP was cleaved with the restriction enzymes SphI and EcoRI, blunt treated, subsequently the SP-BFP fragment was cleaved and cloned into a site where the pUC19-VGF vector (International Publication No. WO2015/076422) was cleaved with a restriction enzyme AccI and blunt treated, or cloned into a site where the pUC19-O1L vector (International Publication No. WO2015/076422) was cleaved with a restriction enzyme Xbal and blunt treated, to construct a shuttle vector pTNshuttle/VGF-SP(EL)-BFP or pTNshuttle/O1L-SP(EL)-BFP. Figure 1 shows the structures of the generated plasmids.

For harvesting the recombinant vaccinia virus having the virus genome shown in Figure 2, CV1 cells or RK13 cells cultured at 80% confluent in a 6-well dish were infected with the VGF-/O1L-mitogen-activated protein kinase-dependent recombinant vaccinia virus (International Publication No. WO2015/076422) at MOI = 0.02 to 0.1 and allowed to adsorb at room temperature for 1 hour, subsequently a transfer vector plasmid DNA (pTN-VGF-EL-BFP) mixed with FuGENE HD (Roche) was added to the cells for an uptake in accordance with the manual and the cells were cultured at 37°C for 2 to 5 days. The cells were harvested, frozen, thawed, and then sonicated, suitably diluted, and inoculated to the almost confluent BSC1 cells or RK13 cells, to which Eagle MEM containing 0.8% methyl cellulose and 5% FBS medium were added, and the cells were cultured at 37°C for 2 to 5 days. The medium was removed, the BFP expression plaques were scraped off at the point of a tip and suspended in Opti-MEM medium (Invitrogen). The same operation was further repeated 3 times or more with the BSC1 cells or the RK13 cells to carry out the plaque purification. The suspension of plaque collected after the plaque purification was sonicated, subsequently the genome DNA was extracted from 200 µL thereof in accordance with the manual using High Pure Viral Nucleic Acid Kit (Roche) and screened by PCR. PCR was carried out on VGF using two primers (SEQ ID NO: 3 and SEQ ID NO: 4) and on OIL using two primers (SEQ ID NO: 5 and SEQ ID NO: 6), whereby the clones in which PCR product of a predetermined size was detected were confirmed for the nucleotide sequence of the PCR product by the direct sequence. The virus clones LC16mO VGF-EL-BFP/O1L-p7.5E-DsRed which had no problem in the nucleotide sequence were selected and amplified in the A549 cells or the RK13 cells, subsequently measured for the virus titer in the RK13 cells, and subjected to the experiment as the platform for generating the gene recombinant vaccinia.

### Example 2 Generation of gene recombinant vaccinia

For inserting an expression unit of a foreign gene to the VGF gene, a yeast cytosine deaminase (CD) / uracil phosphoribosyltransferase (UPRT) fusion (Fcy::Fur) gene region was amplified using two primers (SEQ ID NO: 7 and SEQ ID NO: 8) using the plasmid DNA of pORF5-Fcy::Fur (InvivoGen) as a template. The PCR product thereof was cleaved with the restriction enzymes Nhel and Mlul and cloned into the same restriction enzyme sites of the pIRES vector (Clontech) to construct pIRES-Fcy::Fur. The Fcy::Fur obtained by cleaving the pIRES-Fcy::Fur with the restriction enzymes Sfil and SphI was cloned into the same restriction enzyme sites of the pTK-SP-BFP vector in such a way as to be replaced with the BFP gene, whereby pTK-SP-Fcy::Fur was constructed. On the other hand, the *E. coli* LacZ gene (SEQ ID NO: 9) synthesized in such a way as to be codon-optimized for *H. sapiens* was cleaved with the restriction enzymes Agel and Nhel and cloned into the same restriction enzyme sites of the pTNshuttle/VGF-SP(EL)-BFP vector in such a way as to be replaced with the BFP gene, whereby pTNshuttle/VGF-SP(EL)-LacZ was constructed. Subsequently, the Fcy::Fur fragment obtained by cleaving pTK-SP-Fcy::Fur with the restriction enzyme SphI, blunt treating, subsequently cleaving with Agel was cloned into the vector fragment obtained by cleaving pTNshuttle/VGF-SP(EL)-BFP with Nhel, blunt treating, and subsequently cleaving with Agel, whereby pTNshuttle/VGF-SP(EL)-Fcy::Fur was constructed.

For inserting an expression unit of a foreign gene into the OIL gene, the LucGFP fragment obtained by cleaving pIRES-LucGFP (International Publication No. WO2015/076422) with the restriction enzyme EcoRI, blunt treating, and subsequently cleaving with EcoRI was cloned into the vector fragment obtained by cleaving pTNshuttle/O1L-SP(EL)-BFP with the restriction enzymes Agel and Nhel and blunt treating, whereby pTNshuttle/O1L-SP(EL)-LucGFP was constructed. Additionally, the *E. coli* LacZ gene (SEQ ID NO: 9) synthesized in such a way as to be codon-optimized for *H. sapiens* was cleaved with the restriction enzymes Agel and Nhel and cloned into the same restriction enzyme sites of the pTNshuttle/O1L-SP(EL)-BFP vector in such a way as to be replaced with the BFP gene, whereby pTNshuttle/O1L-SP(EL)-LacZ was constructed.

For harvesting the recombinant vaccinia virus having the virus genome shown in Figure 2, CV1 cells cultured at 80% confluent in a 6-well dish were infected with the vaccinia virus (LC16mO VGF-EL-BFP/O1L-p7.5E-DsRed) at MOI = 0.02 to 0.1 and allowed to adsorb at room temperature for 1 hour, subsequently a transfer vector plasmid DNA (pTNshuttle/VGF-SP(EL)-Fcy::Fur) mixed with FuGENE HD (Roche) was added to the cells for an uptake in accordance with the manual and the cells were cultured at 37°C for 2 to 5 days. The cells were harvested, frozen, thawed, and then sonicated, suitably diluted, and inoculated to the almost confluent BSC1 cells, to which Eagle MEM containing 0.8% methyl cellulose and 5% FBS medium were added, and the cells were cultured at 37°C for 2 to 5 days. The medium was removed, the plaques in which the BFP expression was lost were scraped off at the point of a tip and suspended in Opti-MEM medium (Invitrogen). The same operation was further repeated 3 times or more with the BSC1 cells to carry out the plaque purification. The suspension of plaque collected after the plaque purification was sonicated, subsequently the genome DNA was extracted from 200 µL thereof in accordance with the manual using High Pure Viral Nucleic Acid Kit (Roche) and screened by PCR. PCR was carried out on the VGF region using two primers in the same manner as described above, whereby the clones in which PCR product of a predetermined size was detected were confirmed for the nucleotide sequence of the PCR product by the direct sequence. The virus clones LC16mO VGF-EL-Fcy::Fur/O1L-p7.5E-DsRed which do not have any problem in the nucleotide sequence were selected and amplified in the A549 cells, subsequently measured for the virus titer in the RK13 cells, and subjected to the experiment.

For harvesting the recombinant vaccinia virus having the virus genome shown in Figure 3, the recombinant viruses were harvested by the same method as above, using the DsRed expression loss as the indicator, based on the vaccinia virus (LC16mO VGF-EL-Fcy::Fur/O1L-p7.5E-DsRed) and the transfer vector plasmid DNA (pTNshuttle/O1L-SP(EL)-LucGFP, or pTNshuttle/O1L-SP(EL)-LacZ) and thereby defined as LC16mO VGF-EL-Fcy::Fur/O1L-EL-LucGFP or LC16mO VGF-EL-Fcy::Fur/O1L-EL-LacZ. Each recombinant virus, after mass-cultured in A549 cells and purified, was measured for the virus titer in the RK13 cells and subjected to the experiment.

As described above, the present rescue system can generate the recombinant vaccinia viruses expressing only 2 types of foreign genes of interest based on the platform for generating the gene recombinant vaccinia expressing 2 different types of fluorescent proteins (Figure 4).

### Example 3 Characterization of gene recombinant vaccinia

For confirming the actual expression and function of the foreign genes introduced by the present rescue system, characterization of the generated recombinant vaccinia viruses was carried out. First, for confirming the expressions of the LucGFP and LacZ genes inserted into the OIL region, human pancreatic cancer Panc1 cells were inoculated at 6×10³/well in a 96-well plate, cultured at 37°C for 24 hours, and subsequently infected with LC16mO VGF-EL-Fcy::Fur/O1L-EL-LucGFP or LC16mO VGF-EL-Fcy::Fur/O1L-EL-LacZ at MOI = 0, 0.01, 0.1 respectively (n=3), followed by, after 72 hours from the infection, observing the GFP fluorescence, quantifying the luciferase emission, X-Gal Staining, and measuring titers (Figures 5 to 9). When the cells infected with LC16mO VGF-EL-Fcy::Fur/O1L-EL-LucGFP were photographed using a fluorescence microscope (BZ-X700, KEYENCE CORPORATION), the expression of the GFP gene integrated to the viruses was observed as green fluorescence and the fluorescence regions were confirmed to have expanded in proportion with the virus multiplicity of infection (Figure 5). Further, when the GFP fluorescence intensity of the virus was quantified by the hybrid cell count in BZ-X700 (KEYENCE CORPORATION), the GFP fluorescence intensity was confirmed to have been enhanced in proportion with the increase in the virus multiplicity of infection in consistency with the observed images (Figure 6). Furthermore, when the expression of the luciferase gene was quantified in terms of the emission using Promega One-Glo Luciferase Assay System (Promega Corporation), the increase in the emission was confirmed in proportion with the virus multiplicity of infection as in the observed images and GFP fluorescence (Figure 7).

Furthermore, staining of the cells infected with LC16mO VGF-EL-Fcy: :Fur/O1L-EL-LacZ was carried out using Genlatis X-Gal Staining Assay Kit (Genlantis Inc.) (Figure 8). The virus infected region expressing LacZ was stained blue by the X-Gal Staining, and further the expansion of the stained region associated with the increase in the virus multiplicity of infection was confirmed.

Additionally, when cell-associated virus was harvested from the Panc1 cells infected with the above LC16mO VGF-EL-Fcy::Fur/O1L-EL-LucGFP or LC16mO VGF-EL-Fcy::Fur /OIL-EL-LacZ by freezing and thawing, cell disrupting (sonicating) treatment, and centrifuging (2,000 rpm, 4°C, 10 min) and the titer was measured using the RK13 cells, both viruses were confirmed to have increased virus titers associated with the increase in the virus multiplicity of infection (Figure 9), revealing the consistent tendency with the enhanced GFP fluorescence intensity, the increased luciferase emission, and the expanded X-Gal stained region described above.

Finally, for analyzing the expression of the Fcy::Fur gene introduced into the VGF region, seven human pancreatic cancer cell lines (AsPC1·AsPC1 CD44v9 High: 7x10³/well, BxPC3·Panc1·SW1990: 6x10³/well, MiaPaca2: 5x10³/well, and Panc10.05: 1x10⁴/well), eight human ovarian cancer cell lines (A2780: 6x10³/well, ES-2: 6x10³/well, KF: 6x10³/well, KFTx: 6x10³/well, OVCAR3: 5x10³/well, RMG-1: 6x10³/well, SHIN-3: 6x10³/well, and SKOV3: 6x10³/well), four human colon cancer cell lines (Caco2, HT29, Lovo, and SW480 (all at 6x10³/well)), two human breast cancer cell lines (MCF-7 and SK-BR-3 (all at 6x10³/well)), and one human lung cancer cell line (A549: 6x10³/well) were inoculated in a 96-well plate, cultured at 37°C for 24 hours, and subsequently infected with three viruses, VGF-/O1L-(International Publication No. WO2015/076422) (LG/DsRed), LC16mO VGF-EL-Fcy::Fur/O1L-EL-LucGFP (FF/LG), or LC16mO VGF-EL-Fcy::Fur/O1L-EL-LacZ (FF/LacZ) at MOI = 0.01, 0.1, 1 respectively (n=3). Then, after 48 hours from the infection, 5-FC was added at 0 µg/ml, 1 µg/ml, 10 µg/ml, 100 µg/ml, cultured at 37°C for 72 hours, and subsequently measured for the cell viabilities using Promega Celltiter 96 AQueous NonRadioactive Cell Proliferation Assay (Promega Corporation) (Figure 10-1 to Figure 10-18).

At MOI = 1 where a virus multiplicity of infection is high, for many cell lines such as pancreatic cancers AsPC1 CD44v9 High, BxPC3, MiaPaca2, Panc10.05, Panc1, and SW1990, ovarian cancers ES-2, KF, KFTx, RMG-1, and SHIN-3, colon cancers Caco2 and SW480, breast cancers MCF-7 and SK-BR-3, and lung cancer A549 which are killed by the virus alone, enhanced cell death due to the addition of 5-FC was not found, whereas for pancreatic cancer AsPC1, ovarian cancers A2780, OVCAR3, and SKOV3, colon cancers HT-29 and Lovo, and so on in which the cell death is hardly caused by virus alone, the enhanced cell death depending on the amount of 5-FC added was confirmed (Figure 10-1, Figure 10-2, Figure 10-7, Figure 10-8, Figure 10-13, and Figure 10-16). At MOI = 1, for the cell lines such as pancreatic cancers AsPC1 CD44v9 High, MiaPaca2, and Panc10.05, ovarian cancers ES-2, KF, KFTx, and SHIN-3, colon cancers Caco2 and SW480, breast cancers MCF-7 and SK-BR-3, and lung cancer A549 in which the cell death was hardly caused by virus alone due to the reduced virus multiplicity of infection, the enhanced cell death was confirmed in the 5-FC amount-dependent manner (Figure 10-3, Figure 10-4, Figure 10-9, Figure 10-10, Figure 10-14, and Figure 10-17). Further, at MOI = 0.01, the 5-FC amount-dependent combination effects were confirmed even in the cell lines such as pancreatic cancers Panc1 and SW1990, and ovarian cancer RMG-1 where the combination effect with 5-FC was not found when virus multiplicity of infection is high (MOI = 1 and MOI = 0.1) (Figure 10-5, Figure 10-6, Figure 10-11, Figure 10-12, Figure 10-15, and Figure 10-18).

### Example 4 Characterization·and study of therapeutic effect of gene recombinant vaccinia in mice model

For confirming the expression and function of the foreign gene introduced by the present rescue system in mice *in vivo,* studies were conducted to detect emission of the luciferase-introduced virus using xenotransplantation models, to examine combined use of 5-FC and the Fcy::Fur-introduced virus using allotransplantation models, and to examine antiviral therapeutic effects in both models.

In the xenotransplantation, six pancreatic cancer cell lines (AsPC1 CD44v9 High, BxPC3, Miapaca2, Panc1, Panc10.05, and SW1990) constantly expressing *Renilla* luciferase (Rluc) were intraperitoneally transplanted at 5x10⁶ cells into the CB.17 ICR-SCID/SCID Jcl mice, and allowed to engraft and grow for 1 to 2 weeks. LC16mO VGF-EL-Fcy::Fur/O1L-EL-LucGFP (FF-LG) was intraperitoneally administered at 1x10⁶PFU to mice having peritoneally disseminated pancreatic cancer after transplantation to confirm the virus growth after administration and the expression of the foreign gene introduced into the virus, and observe the survival rate (Figure 11-1, Figure 11-2, Figure 12-1, Figure 12-2, and Figure 13). The Rluc expression in the tumor cells by the administration of ViviRen *In Vivo Renilla* Luciferase Substrate (Promega Corporation) and the expression of luciferase (Fluc) introduced into the virus by the administration of Vivo Glo Luciferin (Promega Corporation) were respectively detected noninvasively in terms of the emission. *In vivo* imaging system (Berthold, NightDHADE LB985) was used for the emission detection and the quantification of emission value.

Observing the expression of the virus Fluc, intraperitoneal intense luciferase emission (≈ virus growth) was confirmed in all the virus administered mice (FF-LG) 2 days after virus administration, but the emission was attenuated 10 days after virus administration (Figure 11-1). Further, observing the expression of the tumor Rluc, intraperitoneal intense Rluc emission (≈ tumor growth) was confirmed in all the mice until 2 days before administration but only the virus nonadministered mice (PBS) retained intense Rluc emission 11 days after virus administration and the intraperitoneal Rluc expression of the virus administered mice (FF-LG) was notably attenuated (Figure 11-2). From the quantified results of Fluc and Rluc, the emission value of Fluc in the virus administered mice was reduced from 2 to 10 days after administration (Figure 12-1). Further, the emission value of Rluc increased in the virus nonadministered mice from before administration to 11 days after administration but notably reduced in the virus administered mice from before administration to 11 days after administration (Figure 12-2). Particularly, the Rluc emission value of the virus administered mice on day 11 after administration was attenuated to about 1/10 to 1/100 of the virus nonadministered mice. Additionally, observing the survival rate after virus administration, the virus administered mice had extended survival durations compared to the virus nonadministered mice in all the pancreatic cancer cell transplanted models (Figure 13).

Further, in the allotransplantation, mouse colon cancer MC38 cells were subcutaneously transplanted at 1x10⁵ cells to C57BL6Jjcl mice and allowed to grow for about 10 days until the tumor reached 44 to 95 mm³. After the tumor was grown, LC16mO VGF-EL-Fcy::Fur/O1L-EL-LacZ(FF/LacZ) was directly administered at 1x10⁸ PFU into the tumor, and 1 day after, 12.5 mg of 5-FC was intraperitoneally administered to the mice. The alternate administration of the virus and ·5-FC was carried out 6 times, and the antitumor effect and the combination effect with the prodrug of the virus were studied by subsequent tumor size measurement. As a result, the combination use of the virus and prodrug (FF-LacZ+5-FC) suppressed the growth of the tumor compared to the virus nonadministered mice (PBS+PBS, PBS+5-FC) and virus single-administered mice (FF-LacZ+PBS), and the two-way ANOVA analysis of variance confirmed that there was a significant difference (* P<0.05) compared to the tumor volume of the virus nonadministered mice (PBS+PBS) 15 days later and there was an extremely significant difference compared to the tumor volumes of the virus nonadministered mice and the virus single-administered mice 17 days later (*** P<0.001, **** P<0.0001) (Figure 14).

As stated above, the recombinant vaccinia viruses wherein a foreign gene is introduced by the present rescue system are confirmed to have expressed the foreign gene associated with the virus growth in the infected cells and acquired the character in accordance with the gene function.

### Industrial Applicability

The gene expressing a foreign gene prepared by the method of the present invention can be used for the treatment of various diseases.

### Sequence List Free Text

SEQ ID NOS: 1 to 8 primers
SEQ ID NO: 9 synthetic

## Claims

1. A vaccinia virus, having suicide genes, cytosine deaminase (CD) gene and uracil phosphoribosyltransferase (UPRT) gene, inserted into one of the vaccinia virus growth factor (VGF) and the OIL gene, having a marker gene inserted into the other of the vaccinia virus growth factor (VGF) and the OIL gene, being deprived of functions of the vaccinia virus growth factor (VGF) and the OIL gene, not growing in normal cells and growing specifically in cancer cells, and having oncolytic effect damaging specifically cancer cells; and further killing cancer cells by the action of the suicide genes.

2. The vaccinia virus according to claim 1, which is LC16 strain or LC16mO strain.

3. The vaccinia virus according to claim 1 or 2 for use in treating cancer.

4. The vaccinia virus for use according to claim 3 used in combination with 5-fluorocytosine (5-FC) or 5-fluorouracil (5-FU).

5. A method for introducing a foreign gene to a vaccinia virus, comprising inserting a marker gene into an endogenous gene or an untranslated region of the vaccinia virus by introducing into the vaccinia virus a plasmid for marker gene insertion having a structure wherein the marker gene is inserted into DNA encoding the endogenous gene or DNA of the untranslated region of the vaccinia virus into which the marker gene is to be inserted and inserting the marker gene into the endogenous gene or the untranslated region of the vaccinia virus by homologous recombination; and further replacing the marker gene inserted into the endogenous gene or the untranslated region with a different foreign gene, wherein it is determined that the foreign gene has been replaced with the marker gene and introduced into the vaccinia virus, using a loss of a signal caused by the marker gene as an indicator.

6. A method for introducing a foreign gene into a vaccinia virus, comprising replacing a marker gene in an endogenous gene or an untranslated region of the vaccinia virus into which the marker gene has been inserted in advance with a different foreign gene, wherein it is determined that the foreign gene has been replaced with the marker gene and introduced into the vaccinia virus, using a loss of a signal caused by the marker gene as an indicator.

7. The method according to claim 5 or 6, wherein the vaccinia virus is LC16 strain or LC16mO strain.

8. The method according to any one of claims 5 to 7, wherein the marker gene is selected from the group consisting of luciferase (LUC) gene, fluorescent protein gene, β-glucuronidase (GUS) gene, chloramphenicol acetyl transferase (CAT) gene, and β-galactosidase (LacZ) gene.

9. The method according to any one of claims 5 to 8, wherein the endogenous gene into which the marker gene has been inserted is selected from the group consisting of hemagglutinin (HA) gene, thymidine kinase (TK) gene, vaccinia virus growth factor (VGF) gene, and OIL gene.

10. The method according to any one of claims 6 to 9, wherein the foreign gene to be introduced is a therapeutic gene having a cytotoxic effect or an immunostimulatory effect, or DNA encoding an antigen of a cancer, a virus, a bacterium, or a protozoan, or a suicide gene.

11. The method according to claim 10, wherein the foreign gene is selected from the group consisting of cytosine deaminase (CD) gene, uracil phosphoribosyltransferase (UPRT) gene, and herpes simplex virus thymidine kinase (HSV-tk) gene.

## Patentansprüche

1. Vaccinia Virus, aufweisend Suizid-Gene, Cytosin-Desaminase-Gen (CD-Gen) und Uracil-Phosphoribosyl-Transferase-Gen (UPRT-Gen), eingefügt in eines von dem Vaccinia Virus-Wachstumsfaktor-Gen (VGF-Gen) und dem O1L-Gen, aufweisend ein Marker-Gen, eingefügt in das andere von dem Vaccinia Virus-Wachstumsfaktor-Gen (VGF-Gen) und dem O1L-Gen, der Funktionen des Vaccinia Virus-Wachstumsfaktor-Gens (VGF-Gens) und des O1L-Gens beraubt, nicht in normalen Zellen wachsend und spezifisch in Krebszellen wachsend, und eine onkolytische Wirkung, die Krebszellen spezifisch schädigt, aufweisend; und weiterhin Krebszellen abtötend durch die Wirkung der Suizid-Gene.

2. Vaccinia Virus nach Anspruch 1, das Stamm LC16 oder Stamm LC16mO ist.

3. Vaccinia Virus nach Anspruch 1 oder 2 zur Verwendung bei der Behandlung von Krebs.

4. Vaccinia Virus zur Verwendung nach Anspruch 3, verwendet in Kombination mit 5-Fluorcytosin (5-FC) oder 5-Fluoruracil (5-FU).

5. Verfahren zum Einbringen eines fremden Gens in ein Vaccinia Virus, umfassend das Einfügen eines Marker-Gens in ein endogenes Gen oder eine untranslatierte Region des Vaccinia Virus, indem in das Vaccinia Virus ein Plasmid für die Einfügung des Marker-Gens eingebracht wird, mit einer Struktur, wobei das Marker-Gen eingefügt wird in die DNA, die das endogene Gen codiert, oder die DNA der untranslatierten Region des Vaccinia Virus, in welche(s) das Marker-Gen eingefügt werden soll, und das Einfügen des Marker-Gens in das endogene Gen oder die untranslatierte Region des Vaccinia Virus durch homologe Rekombination; und weiterhin das Ersetzen des in das endogene Gen oder die untranslatierte Region eingefügten Marker-Gens durch ein anderes fremdes Gen, wobei festgestellt wird, dass das fremde Gen durch das Marker-Gen ersetzt und in das Vaccinia Virus eingebracht worden ist, mittels eines Wegfalls eines durch das Marker-Gen als Indikator verursachten Signals.

6. Verfahren zum Einbringen eines fremden Gens in ein Vaccinia Virus, umfassend das Ersetzen eines Marker-Gens in einem endogenen Gen oder einer untranslatierten Region des Vaccinia Virus, in welche(s) das Marker-Gen vorab eingefügt worden ist, durch ein anderes fremdes Gen, wobei festgestellt wird, dass das fremde Gen durch das Marker-Gen ersetzt und in das Vaccinia Virus eingebracht worden ist, mittels eines Wegfalls eines durch das Marker-Gen als Indikator verursachten Signals.

7. Verfahren nach Anspruch 5 oder 6, wobei der Vaccinia Virus Stamm LC16 oder Stamm LC16mO ist.

8. Verfahren nach einem der Ansprüche 5 bis 7, wobei das Marker-Gen ausgewählt ist aus der Gruppe bestehend aus Luciferase-Gen (LUC-Gen), Gen eines fluoreszierenden Proteins, β-Glucuronidase-Gen (GUS-Gen), Chloramphenicol-AcetylTransferase-Gen (CAT-Gen) und β-Galactosidase-Gen (LacZ-Gen).

9. Verfahren nach einem der Ansprüche 5 bis 8, wobei das endogene Gen, in das das Marker-Gen eingefügt worden ist, ausgewählt ist aus der Gruppe bestehend aus Hämagglutinin-Gen (HA-Gen), Thymidin-Kinase-Gen (TK-Gen), Vaccinia Virus-Wachstumsfaktor-Gen (VGF-Gen) und O1L-Gen.

10. Verfahren nach einem der Ansprüche 6 bis 9, wobei das einzubringende fremde Gen ein therapeutisches Gen mit einer zytotoxischen Wirkung oder einer immunstimulierenden Wirkung, oder DNA, die ein Antigen eines Karzinoms, eines Virus, eines Bakteriums oder eines Protozoons codiert, oder ein Suizid-Gen ist.

11. Verfahren nach Anspruch 10, wobei das fremde Gen ausgewählt ist aus der Gruppe bestehend aus Cytosin-Desaminase-Gen (CD-Gen), Uracil-Phosphoribosyl-Transferase-Gen (UPRT-Gen) und Herpes simplex Virus-Thymidin-Kinase-Gen (HSV-tk-Gen).

## Revendications

1. Virus de la vaccine, ayant des gènes suicide, le gène de la cytosine désaminase (CD) et le gène de l'uracile phosphoribosyltransférase (UPRT), insérés dans un du gène du facteur de croissance du virus de la vaccine (VGF) et du gène O1L, ayant un gène marqueur inséré dans l'autre du gène du facteur de croissance du virus de la vaccine (VGF) et du gène O1L, étant dépourvu de fonctions du gène de facteur de croissance du virus de la vaccine (VGF) et du gène O1L, ne croissant pas dans le cellules normales et croissant spécifiquement dans les cellules cancéreuses, et ayant un effet oncolytique d'endommagement spécifiquement des cellules cancéreuses ; et tuant en outre les cellules cancéreuses par l'action des gènes suicide.

2. Virus de la vaccine selon la revendication 1, qui est la souche LC16 ou la souche LC16mO.

3. Virus de la vaccine selon la revendication 1 ou 2 destiné à être utilisé dans le traitement du cancer.

4. Virus de la vaccine destiné à être utilisé selon la revendication 3 utilisé en combinaison avec la 5-fluorocytosine (5-FC) ou le 5-fluorouracile (5-FU).

5. Procédé d'introduction d'un gène étranger dans un virus de la vaccine, comprenant l'insertion d'un gène marqueur dans un gène endogène ou une région non traduite du virus de la vaccine, en introduisant dans le virus de la vaccine un plasmide pour l'insertion d'un gène marqueur ayant une structure dans laquelle le gène marqueur est inséré dans l'ADN codant pour le gène endogène ou l'ADN de la région non traduite du virus de la vaccine dans laquelle le gène marqueur doit être inséré et l'insertion du gène marqueur dans le gène endogène ou la région non traduite du virus de la vaccine par recombinaison homologue ; et en outre en remplaçant le gène marqueur inséré dans le gène endogène ou la région non traduite avec un gène étranger différent, étant déterminé que le gène étranger a été remplacé avec le gène marqueur et introduit dans le virus de la vaccine, en utilisant une perte d'un signal provoqué par le gène marqueur comme indicateur.

6. Procédé d'introduction d'un gène étranger dans un virus de la vaccine, comprenant le remplacement d'un gène marqueur dans un gène endogène ou une région non traduite du virus de la vaccine dans lequel le gène marqueur a été inséré à l'avance avec un gène étranger différent, étant déterminé que le gène étranger a été remplacé avec le gène marqueur et introduit dans le virus de la vaccine, en utilisant une perte de signal provoqué par le gène marqueur comme indicateur.

7. Procédé selon la revendication 5 ou 6, dans lequel le virus de la vaccine est la souche LC16 ou la souche LC16mO.

8. Procédé selon l'une quelconque des revendications 5 à 7, dans lequel le gène marqueur est choisi dans le groupe constitué par le gène de la luciférase (LUC), un gène de protéine fluorescente, le gène de la β-glucuronidase (GUS), le gène de la chloramphénicol acétyltransférase (CAT) et le gène de la β-galactosidase (LacZ).

9. Procédé selon l'une quelconque des revendications 5 à 8, dans lequel le gène endogène dans lequel le gène marqueur a été inséré est choisi dans le groupe constitué par le gène de l'hémagglutinine (HA), le gène de la thymidine kinase (TK), le gène du facteur de croissance du virus de la vaccine (VGF) et le gène O1L.

10. Procédé selon l'une quelconque des revendications 6 à 9, dans lequel le gène étranger à introduire est un gène thérapeutique ayant un effet cytotoxique ou un effet immunostimulateur, ou un ADN codant pour un antigène d'un cancer, d'un virus, d'une bactérie ou d'un protozoaire ou un gène suicide.

11. Procédé selon la revendication 10, dans lequel le gène étranger est choisi dans le groupe constitué par le gène de la cytosine désaminase (CD), le gène de l'uracile phosphoribosyltransférase (UPRT) et le gène de la thymidine kinase du virus de l'herpès simplex (HSV-tk).
